# EUROPEAN PATENT APPLICATION

(11) **EP 4 091 625 A1**
(43) Date of publication of application: **23.11.2022**
(21) Application number: 21175483.3
(22) Date of filing: 22.05.2021
(51) Int. Cl.: A61K 38/26, A61K 38/22, A61P 3/04, A61P 3/10, A61P 25/28

(54) **COMPOSITIONS COMPRISING SHORT-ACTING HORMONES FOR TREATING OR PREVENTING OBESITY AND PUMPS COMPRISING SAID COMPOSITION**

(71) Applicant: Adocia, 69003 Lyon (FR)
(72) Inventor: The designation of the inventor has not yet been filed
(74) Representative: Tripoz, Inès

(57) **Abstract**

The invention relates to therapies for treating obesity and overweight.

The invention is about compositions comprising at least 2 short-acting peptides chosen in at least two of the following groups, GLP-1 receptor agonist (abbreviated RA), Amylin RA and Glucagon RA, schemes of administration of such compositions and devices comprising such a composition.

## Description

The invention relates to therapies for treating obesity and overweight.

The invention is about compositions comprising at least 2 short-acting peptides chosen in at least two of the following groups, GLP-1 receptor agonist (abbreviated RA), Amylin RA and Glucagon RA, schemes of administration of such compositions and devices comprising such a composition.

In recent times, obesity has spread rapidly in the world, with the US and China being the most affected population. Today, 650 million of adults (13% of adults) and about 124 million of children older than 5 are obese in the world. In the US, 42.8% of adults and 18.5% of children are obese.

It has major health implications that can cause premature death due the increased risk of developing several diseases including osteoarthtitis, non-alcoholic fatty liver disease, type II diabetes, cerebrovascular disease, sleep apnea and asthma.

A weight loss as small as 5 % is thought to significantly reduce the incidence of at least some severe complications.

As seen above, obesity has a high prevalence worldwide and induces a high risk of associated disease. However, few treatment options are available, including pharmacotherapy.

To treat this disease, therapies have focused on reducing food intake and increasing energy expenditure in patients.

A first proposed therapy consists in a surgical solution for reducing the gastric volume. The gastric volume reduction procedures are generally based on the provision of vertical and/or horizontal gastroplasty in which sutures, staples or other fixations are used to join opposing surfaces of the stomach in order to create a reduced volume pouch and thereby reduce caloric intake.

However, such surgical solution is irreversible, strongly invasive, and may lead to heavy side effects and long-term risks.

A second proposed therapy consists in implantable devices.

One implantable solution is based on the provision of a gastric banding. Such banding creates an upper chamber above the band to produce a sensation of satiety after consuming only a small volume of food. However, the provision of gastric banding does not address all mechanisms associated with obesity, for example with regard to patients continuously eating small volumes of high caloric food or consuming high caloric liquid foods.

Another implantable solution is based on the placement of an intra-gastric balloon within the stomach. However, some studies showed modest weight loss, and often a diminution of the effects of these balloons after three to four weeks.

The most promising therapies relies on pharmacotherapy. Recently, use of long acting hormones have yielded viable options to induce weight loss in obese patients.

The use of hormones with long duration of action is a well known paradigm because the necessity of frequent injections reduces patients long term adherence to the treatment. Thus a treatment with long-acting drugs, in particular with once-weekly injections, is the only proposed way today to convince patients to take such a treatment and also to continue this treatment over time.

Hormone therapies in development today, such as Semaglutide and Cagrilintide (from Novo Nordisk), Tirzepatide (from Eli Lilly) or Glucagon-GLP-1 dual agonists (from Boehringer Ingelheim and Zealand), are some examples of this strategy.

Furthermore, clinical results published with these classes of hormones have shown a significant efficacy, leading to the development of more products of these classes.

However, these classes of hormones induce side effects such as nausea, vomiting or diarrheas.

These side effects are a major drawback as by design, long acting drugs remain in circulation for a long duration (e.g. more than a week) without the possibility to end their side effects quickly when they occur. In this case, the patient has no other choice than to wait for the drug concentration to drop below the level inducing unwanted side effects. Thus the side effect lasts for a long time, such as one or several days.

Another issue with this long acting strategy is that the patient cannot decide to take a short break for social events, such as a family meal, or a day off treatment to release pressure, which could contribute to a better acceptation of the treatment in the long term and limit drop-out rates.

Another drawback of these long acting hormone treatments is that they cannot be modulated with a daily titration schedule to optimize efficacy and tolerance. It is well known that the concentration of the long acting hormones changes during the week with a difference of more than 30% between the peak and valley. Concentrations may then be too low at times to induce the best effect and too high at other times to provide the best tolerance.

All these drawbacks are significantly limiting their long term treatment adoption by patients and doctors, , as demonstrated by the significant percentage of patient drop-out, in particular due to side effects. For example it was reported that with a once-a-week long acting GLP-1 RA treatment, 46.7% of type 2 diabetic patients stopped after less then one year.

An aim of the present invention is to provide a composition, a therapeutical scheme and a device to help patients efficiently lose weight by giving them the possibility to control the dosing in order to remedy to at least one of the aforementioned drawbacks. More particularly, an aim of the present invention is to provide a device allowing a continuous delivery of the composition while limiting unease and discomfort sensations, thus improving compliance, and/or improving the performance of the treatment.

The invention proposes a way to solve at least part of the the problems caused by the current treatment of overweight, obesity and related disease. This goes against the path used by the main actors of the field as it involves short-acting active principles while the main players of the field believe that the best solution involves long-acting active principles.

The therapeutical scheme according to the invention offers to patients suffering from overweight, obesity or related disease a way to control the hormones side effects by a patient driven control of the treatment administration. Thus the patient can temporarily stop or diminish the delivery of their treatment until the side-effect(s) disappear(s) and then resume or increase the dose delivery of the treatment, without compromising the long-term efficacy of the treatment or inducing treatment discontinuation.

The therapeutical scheme comprising compositions according to the invention may lead to:
- a better control of undesirable side effects by the patient and improvement of the compliance,
- a better shaping of the treatment anda better efficiency per molecule used.

In summary, an aim of the invention is to offer patient:
- to confer the advantages of long-acting hormones in that it maintains an efficient plasmatic concentrations of the hormones over time, and
- to limit the undesirable side effects related to the use of long-acting hormones, as the side effects can be managed, controlled or even stopped, relatively quickly by the patients, and
- to decrease, or even stop, momentarily the treatment for a while allowing him to take a break, thus leading to an improved compliance in the long term.

Thus the invention allows the patient to self adjust his treatment, on the contrary to the current long acting hormones treatments where the patient is passive, and can only choose to discontinue their treatment or carry on as is.

These advantages may be reached with the use of a device, such as a simple pump, in particular a subcutaneous infusion pump, for example an insulin pump, with a composition as disclosed below.

The invention relates to a composition comprising at least two short-acting peptides chosen in at least two of the following groups, short-acting GLP-1 RA, short-acting amylin RA and short-acting glucagon RA.

It also relates to a composition comprising at least two short-acting peptides chosen in at least two of the following groups, short-acting GLP-1 RA, short-acting amylin RA and short-acting glucagon RA for use as a medicament.

It also relates to a composition comprising at least two short-acting peptides chosen in at least two of the following groups, short-acting GLP-1 RA, short-acting amylin RA and short-acting glucagon RA for use in a method of treating overweight, obesity, and related disease.

It also relates to a composition comprising at least two short-acting peptides chosen in at least two of the following groups, short-acting GLP-1 RA, short-acting amylin RA and short-acting glucagon RA for use in a method of preventing overweight, obesity, and related disease.

By "preventing or treating overweight, obesity and related diseases" is meant preventing or treating obesity, NASH, NAFLD, overweight, reducing body weight and/or food intake, or inducing energy expenditure or satiety.

In another embodiment, the composition is for use in a method for preventing or treating neurodegenerative diseases.

In another embodiment, the composition is used for preventing or treating Type 2 Diabetes.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes, characterised in that the composition is administered in a therapeutical scheme that comprises modulation of the delivered dose, such as increasing and decreasing the delivery, or even stop and restart periods.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes,characterised in that the composition is administered in a therapeutical scheme that comprises modulation of the delivered dose, such as increasing and decreasing the delivery, or even stop and restart periods.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes, characterised in that the composition is administered in a therapeutical scheme that further comprises modulation periods that allow a modulation of the daily dose, for example from day to day.

It further relates to a composition comprising at least two short-acting peptides chosen in at least two of the following groups, short-acting GLP-1 RA, short-acting amylin RA and short-acting glucagon RA, for use in a method of preventing or treating overweight, obesity, and related diseases and/or neurodegenerative diseases and /or Type 2 Diabetes, characterised in that the composition is administered in a therapeutical scheme that further comprises modulation periods that allow a modulation of the rate of delivery intraday.

By intraday is meant a period going from 0h00 to 24h00.

It further relates to a composition comprising at least two short-acting peptides chosen in at least two of the following groups, short-acting GLP-1 RA, short-acting amylin RA and short-acting glucagon RA for use in a method of preventing or treating overweight, obesity, and related diseases and/or neurodegenerative diseases and /or Type 2 Diabetes, characterised in that the composition is administered in a therapeutical scheme that further comprises modulation periods that allow a decrease of the daily dose until it is temporarily discontinued.

By "temporarily" is meant periods such as 1h, 2h, 3h, 6h, 8h, 12h, 24h, 2 days, 4 days or even one week, 2 weeks or a month.

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the composition is administered in a therapeutical scheme that comprises stop and restart times.

Stop and restart time may be the same as defined for the term "temporarily".

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the composition is administered in a therapeutical scheme that comprises modulation of the delivered dose.

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the composition is administered in a therapeutical scheme that comprises modulation of the delivered dose during the day without changing the total daily dose.

It further relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that the molar ratio of short-acting GLP-1 RA and short-acting glucagon RA in the composition is such that it leads to a molar ratio of short-acting GLP-1 RA and short-acting glucagon RA at steady state is comprised betweeen 0.1 to 15.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that amylin RA is pramlintide administered at a daily dose comprised from 150 to 2000 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that amylin RA is pramlintide administered at a daily dose comprised from 500 to 2000 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is exenatide administered at a daily dose comprised from 10 to 250 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is exenatide administered at a daily dose comprised from 60 to 180 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is lixisenatide administered at a daily dose comprised from 20 to 600 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that GLP-1 RA is lisixenatide administered at a daily dose comprised from 120 to 360 µg/day.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in that Glucagon RA is human glucagon administered at a daily dose of above 500 µg.

It also relates to a composition comprising at least two short-acting peptides chosen in at least two of the following groups, GLP-1 RA, amylin RA and Glucagon RA for use in a method of preventing or treating overweight, obesity, and related diseases and/or neurodegenerative diseases and /or Type 2 Diabetes, characterised in in that Glucagon RA is dasiglucagon administered at a daily dose of above 250 µg.

The invention also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme that comprises modulation of the delivered dose, such as increasing and decreasing the delivery, or even stop and restart periods.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme that comprises modulation periods that allow a modulation of the daily dose, for example from day to day.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, is administered in a therapeutical scheme that further comprises modulation periods that allow a modulation of the rate of delivery intraday.

By intraday is meant a period going from 0h00 to 24h00.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme comprising modulation periods that allow a decrease of the daily dose until it is temporarily discontinued.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme comprising stop and restart periods.

It further relates to a method of preventing or treating overweight, obesity, and related diseases and/or neurodegenerative diseases and /or Type 2 Diabetes,wherein a composition comprising at least two short-acting peptides chosen in at least two of the following groups, short-acting GLP-1 RA, short-acting amylin RA and short-acting glucagon RA is administered in a therapeutical scheme comprising modulation of the delivered dose.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA is administered in a therapeutical scheme comprising modulation of the delivered dose during the day without changing the total daily dose.

It further relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, the molar ratio of short-acting GLP-1 RA and short-acting glucagon RA in the composition being such that it leads to a molar ratio of short-acting GLP-1 RA and short-acting glucagon RA at steady state is comprised betweeen 0.1 to 15.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that amylin RA is pramlintide administered at a daily dose comprised from 150 to 2000 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that amylin RA is pramlintide administered at a daily dose comprised from 500 to 2000 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is exenatide administered at a daily dose comprised from 10 to 250 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is exenatide administered at a daily dose comprised from 60 to 180 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is lisixenatide administered at a daily dose comprised from 20 to 600 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that GLP-1 RA is lisixenatide administered at a daily dose comprised from 120 to 360 µg/day.

It also relates to a method of preventing or treating overweight, obesity, and related diseases and/or neurodegenerative diseases and /or Type 2 Diabetes, wherein a composition comprising at least two short-acting peptides chosen in at least two of the following groups, short-acting GLP-1 RA, short-acting amylin RA and short-acting glucagon RA, characterised in that Glucagon RA is human glucagon administered at a daily dose of above 500 µg.

It also relates to a method of treating overweight, obesity, and related disease, wherein a composition comprising at least two short-acting peptides chosen in at least two of the following groups, short-acting GLP-1 RA, short-acting amylin RA and short-acting glucagon RA, characterised in in that Glucagon RA is dasiglucagon administered at a daily dose of above 250 µg.

The invention also relates to a device comprising:
- a composition comprising at least two short-acting peptides chosen in at least two of the following groups, short-acting GLP-1 RA, short-acting Amylin RA and short-acting Glucagon RA,
- an administering apparatus for delivering the composition to a patient, said administering apparatus including:
- a reservoir containing the composition,
- a drive mechanism for pumping the composition contained in the reservoir,
- a control unit (24) for controlling the drive mechanism according to an injection profile defining at least one rate for the administration of the composition to the patient,
- an injection set configured to be attached to an infusion site on the patient for the subcutaneous administering of the composition to the patient.

According to an embodiment the device allows the delivery of the composition according to the schemes of administration as defined in the instant specification.

The invention also relates to a composition for use for prevention or treatment of overweight, obesity, and related disease.

The invention also relates to a device comprising a composition for use for prevention or treatment of overweight, obesity, and related disease.

The invention also relates to a method for prevention or treatment of overweight, obesity, and related disease.

The invention relates to an administering device , said device comprises:
- a composition comprising at least two short-acting peptides chosen in at least two of the following groups, GLP-1 RA, Amylin RA and Glucagon RA, for use as a medicament.
- an administering apparatus for delivering the composition to a patient, said administering apparatus including:
- a reservoir containing the composition,
- a drive mechanism for pumping the composition contained in the reservoir,
- a control unit for controlling the drive mechanism according to an injection profile defining at least one rate for the administering of the composition to the patient,
- an injection set configured to be attached to an infusion site on the patient for the subcutaneous administering of the composition to the patient

Preferred, but non-limiting, aspects of the administering device according to the invention are the following:
- device wherein the administering apparatus further comprises an actuator, such as a button, for deactivating the administering apparatus for a predetermined period of time.
- device which further comprises a supervisory terminal for controlling the administering apparatus, said supervisory terminal being configured for adjusting the injection profile according to at least one parameter associated to the patient.
- device wherein the supervisory terminal includes a Graphical User Interface (GUI) for the input by the patient of the at least one parameter associated to the patient.
- device wherein the at least one parameter associated to the patient includes a level of comfort representative of a sensation of nausea felt by the patient, the supervisory terminal being configured for decreasing the at least one rate of the injection profile if said level of comfort does not satisfy a comfort threshold.
- device wherein the at least one parameter associated to the patient includes a level of activity representing whether the patient is:
   ∘ in an active state or
   ∘ in a passive state,
   the supervisory terminal being configured for decreasing the at least one rate of the injection profile if said level of activity corresponds to a passive state of the patient.
   - device wherein the administering apparatus includes at least one sensor, such as a gyroscope and/or an accelerometer, for measuring signals representative of motions of the patient and/or of the orientation of the patient, said signals being transmitted to the control unit and/or the supervisory terminal for detecting whether the patient is in an active state or in a passive state.
   - device wherein the at least one parameter associated to the patient includes an information representative of an event occurring in the life of the patient, the supervisory terminal being configured for decreasing the at least one rate of the injection profile for a duration corresponding to the duration of said event.

The present invention may be more completely understood in consideration of the following detailed description of various embodiments in connection with the accompanying drawings, in which:
- Figure 1 is a schematic perspective view of a device for administering composition 1 ,
- Figure 2 is a block diagram representing an example of an administering apparatus of the device,
- Figure 3 is a schematic view illustrating an example of injection profile for the administration of a composition using the administering apparatus,
- Figure 4 is a schematic view illustrating another example of injection profile for the administration of the composition using the administering apparatus.

Different examples of the device according to the invention will now be described with reference to the figures. In these different figures, equivalent elements are designated by the same numerical reference.

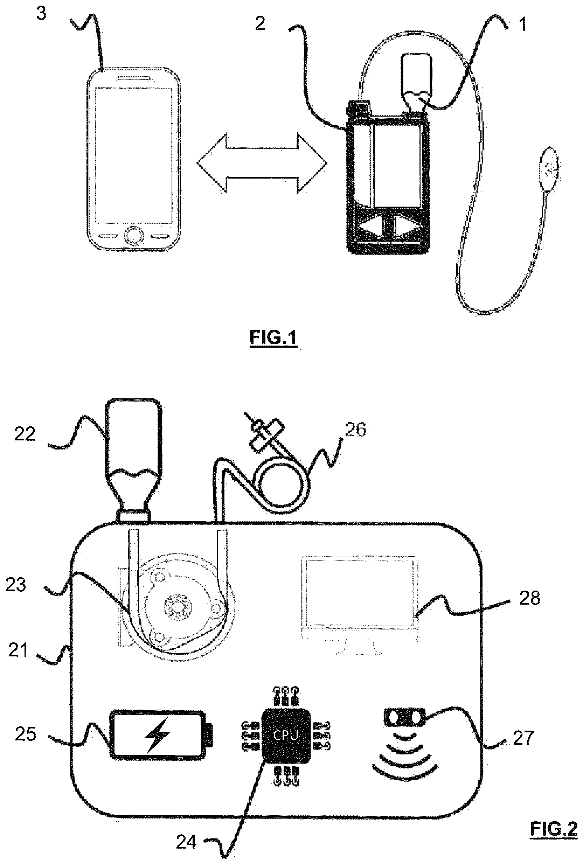

Referring to figure 1, the device comprises:
- a composition 1 such as disclosed above,
- an administering apparatus 2 for delivering the composition 1 to a patient, and
- a supervisory terminal 3 for controlling the administering apparatus 2.

According to an embodiment, the device is configured for a subcutaneous delivery of the composition 1 to a patient.

Preferably, the composition 1 is administered regularly or continuously under the skin of the patient according to an injection profile which can be predetermined, or can be adjustable according to user parameters, as will be described in more details below.

As disclosed in the following description, the device for use in a method for preventing or treating overweight, obesity, and related disease offers the advantages of:
- continuous administration of composition 1,
- precise dosing, and
- programmable delivery schedules.

This results in a closer control and an improved feeling of wellness.

In particular, the device for use in a method for preventing or treating overweight, obesity, and related disease allows administering an amount of composition 1 that is:
- above an efficiency threshold and
- below a discomfort threshold.

This provides the patient with an efficient administration while limiting discomfort sensations.

More precisely the invention relates to a composition comprising two or three short-acting peptides, each chosen from a different group as defined above.

In an embodiment the composition comprises only one compound per group.

In an embodiment the composition does not comprise any other API.

By "active pharmaceutical ingredient" or "API", is meant any substance or combination of substances used in a finished pharmaceutical product (FPP), intended to furnish pharmacological activity or to otherwise have direct effect in the diagnosis, cure, mitigation, treatment or prevention of disease, or to have direct effect in restoring, correcting or modifying physiological functions in human beings."

As used herein, the term "short-acting peptide" refers to a hormone or a peptide that has an apparent half-life of elimination after subcutaneous injection in humans of at least less than 8 hours, in particular less than 5 hours, preferably less than 4 hours or even less than 3 hours.

As used herein, the term "long-acting peptide" refers to a peptide that has an apparent half-life of elimination after subcutaneous injection in humans of more than 8 hours.

In an embodiment, the short-acting peptide is chosen among short-acting GLP-1 RA, short-acting amylin RA or short-acting glucagon RA.

In an embodiment the composition comprises one short-acting GLP-1 RA and one short-acting amylin RA.

In an embodiment the composition comprises one short-acting GLP-1 RA and one short-acting glucagon RA.

In an embodiment the composition comprises one short-acting amylin RA and one short-acting glucagon RA.

In an embodiment the composition comprises one short-acting GLP-1 RA, one short-acting amylin RA and one short-acting glucagon RA.

As used herein, "receptor agonist" means a compound, such as a synthetic peptide, that activates a target receptor and elicits at least one in vivo or in vitro effect elicited by a native agonist for that receptor.

GLP-1 receptor agonist (GLP-1 RA), also known as incretin mimetics, are insulin secretagogues. Among short-acting GLP-1 RA can be cited exenatide and lixisenatide, which are also known for diminishing the food intake and for slowing down the gastric emptying.

Among short-acting amylin RA can be cited pramlintide and salmon calcitonin.

Among short-acting glucagon RA can be cited human glucagon and dasiglucagon.

In an embodiment, the composition comprises at least one short-acting amylin receptor agonist.

By "short-acting" amylin RA is meant an amylin RA that has an apparent half-life of elimination after subcutaneous injection in humans of less than 3 hours, in particular less than 2 hours, preferably less than 1.5 hours or even less than 1 hour.

An agonist of the amylin receptor, or amylin RA, refers to a compound which imitates one or more characteristics of the activity of amylin.

Amylin derivatives are described in the article Yan et al., PNAS, vol. 103, no. 7, p. 2046-2051, 2006.

In an embodiment, the composition is characterized in that the amylin RA is an amylin analog.

As used herein, "analog" means a compound, such as a synthetic peptide, that activates a target receptor and elicits at least one in vivo or in vitro effect elicited by a native agonist for that receptor.

In an embodiment, the amylin RA is chosen in the group consisting of pramlintide and salmon calcitonin.

In an embodiment, the composition is characterized in that the amylin RA is pramlintide (Symlin^{®}) marketed by the company ASTRAZENECA AB.

Pramlintide is an amylin receptor agonist and amylin analog which has been developed by the company Amylin to compensate the lack of physical stability of human amylin. This product, marketed under the name of Symlin^{®}, was approved in 2005 by the FDA for the treatment of type 1 and type 2 diabetes, as a complement to insulin therapy.

Pramlintide is a peptide having the sequence:

### KCNTATCATQRLANFLVHSSNNFGPILPPTNVGSNTY (SEQ ID n°1)

In an embodiment the amylin RA is pramlintide.

In an embodiment, the concentration of pramlintide is ranging from 150 to 10 000 µg/mL.

In an embodiment, the concentration of pramlintide is ranging from 300 to 9 000 µg/mL.

In an embodiment, the concentration of pramlintide is ranging from 500 to 8 000 µg/mL.

In an embodiment, the concentration of pramlintide is ranging from 1000 to 7 000 µg/mL.

In an embodiment, the concentration of pramlintide is ranging from 1000 to 5 000 µg/mL.

In an embodiment, the concentration of pramlintide is ranging from 1000 to 3 000 µg/mL

In an embodiment, the composition allows the delivery of a daily dose of pramlintide ranging from 150 to 2500 µg/day.

In an embodiment, the composition allows the delivery of a daily dose of pramlintide ranging from 250 to 2000 µg/day.

In an embodiment, the composition allows the delivery of a daily dose of pramlintide ranging from 300 to 2000 µg/day.

In an embodiment, the composition allows the delivery of a daily dose of pramlintide ranging from 350 to 2000 µg/day.

In an embodiment, the composition allows the delivery of a daily dose of pramlintide ranging from 400 to 2000 µg/day.

In an embodiment, the composition allows the delivery of a daily dose of pramlintide ranging from 500 to 2000 µg/day.

In an embodiment, the composition comprises at least one short-acting GLP-1 receptor agonist.

By "short-acting" GLP-1 RA is meant a GLP-1 RA that has an apparent half-life of elimination after subcutaneous injection in humans of less than 8 hours, in particular less than 5 hours, preferably less than 4 hours or even less than 3 hours.

In an embodiment, the GLP-1 RA is selected from the group consisting of exenatide (Byetta^{®}, ASTRA-ZENECA), lixisenatide (Lyxumia^{®}, SANOFI), the analogs or derivatives thereof and pharmaceutically acceptable salts thereof.

Exenatide is a peptide having the sequence: HGEGTFTSDL SKQMEEEAVR LFIEWLKNGG PSSGAPPPS (SEQ ID n°2)

Lixisenatide is a peptide having the sequence: HGEGTFTSDL SKQMEEEAVR LFIEWLKNGG PSSGAPPSKK KKKK-[NH2] (SEQ ID n°3)

In one embodiment, the composition comprises at least one short-acting GLP-1 receptor agonist at a concentration ranging from 20 to 3500 µg/ml.

In an embodiment, the composition allows the delivery of a daily dose of short-acting GLP-1 RA ranging from 10 to 600 µg/day.

In one embodiment, the composition comprises exenatide at a concentration ranging from 20 to 1000 µg/ml.

In one embodiment, the composition comprises exenatide at a concentration ranging from 40 to 500 µg/ml

In one embodiment, the composition comprises exenatide at a concentration ranging from 60 to 400 µg/ml.

In one embodiment, the composition comprises exenatide at a concentration ranging from 80 to 300 µg/ml.

In one embodiment, the composition comprises exenatide at a concentration ranging from 90 to 250 µg/ml.

In one embodiment, the composition comprises exenatide at a concentration ranging from 100 to 200 µg/ml.

In one embodiment, the composition comprises exenatide at a concentration ranging from 100 to 1500 µg/ml.

In one embodiment, the composition comprises exenatide at a concentration ranging from 250 to 1200 µg/ml.

In one embodiment, the composition comprises lixisenatide at a concentration ranging from 50 to 2000 µg/ml.

In one embodiment, the composition comprises lixisenatide at a concentration ranging from 100 to 1500 µg/ml.

In one embodiment, the composition comprises lixisenatide at a concentration ranging from 150 to 1000 µg/ml.

In one embodiment, the composition comprises lixisenatide at a concentration ranging from 200 to 750 µg/ml.

In one embodiment, the composition comprises lixisenatide at a concentration ranging from 225 to 650 µg/ml.

In one embodiment, the composition comprises lixisenatide at a concentration ranging from 250 to 500 µg/ml.

In one embodiment, the composition comprises lixisenatide at a concentration ranging from 250 to 3500 µg/ml.

In one embodiment, the composition comprises lixisenatide at a concentration ranging from 500 to 2500 µg/ml.

In an embodiment, the composition allows the delivery of a daily dose of exenatide ranging from 10 to 250 µg/day.

In an embodiment, the composition allows the delivery of a daily dose of exenatide ranging from 20 to 220 µg/day.

In an embodiment, the composition allows the delivery of a daily dose of exenatide ranging from 30 to 210 µg/day.

In an embodiment, the composition allows the delivery of a daily dose of exenatide ranging from 40 to 200 µg/day.

In an embodiment, the composition allows the delivery of a daily dose of exenatide ranging from 50 to 190 µg/day.

In an embodiment, the composition allows the delivery of a daily dose of exenatide ranging from 60 to 180 µg/day.

In an embodiment, the compositions allow the delivery of a daily dose of lixisenatide ranging from 20 to 600 µg/day.

In an embodiment, the compositions allow the delivery of a daily dose of lixisenatide ranging from 40 to 550 µg/day.

In an embodiment, the compositions allow the delivery of a daily dose of lixisenatide ranging from 60 to 500 µg/day.

In an embodiment, the compositions allow the delivery of a daily dose of lixisenatide ranging from 80 to 450 µg/day.

In an embodiment, the compositions allow the delivery of a daily dose of lixisenatide ranging from 100 to 400 µg/day.

In an embodiment, the compositions allow the delivery of a daily dose of lixisenatide ranging from 120 to 360 µg/day.

In an embodiment, the composition comprises at least one short-acting glucagon receptor agonist. By "short-acting" glucagon RA is meant glucagon RA having an apparent half-life of elimination after subcutaneous injection in humans of less than 3 hours, in particular less than 2 hours, preferably less than 1.5 hours or even less than 1 hour.

The glucagon receptor agonist may be a glucagon analog.

As used herein, "analog" means a peptide wherein one or more amino acid residues of the basic human glucagon peptide have been substituted by another amino acid residue and/or wherein one or more amino acid residues of the peptide have been deleted and/or wherein one or more amino acid residues have been added to the peptide. Such addition can take place either at the N-terminal end or at the C-terminal end or both.

The glucagon analogs may present up to 8, in particular up to 7, substitutions, deletions or additions compared to the sequence of human glucagon. The substitutions may be done with standard amino acid residues or by non-standard amino-acid residues, such as synthetic amino acid residues, among which can be cited Aib (2-aminoisobutyric acid).

In an embodiment, the glucagon analog present up to 8, in particular up to 7, substitutions compared to the sequence of human glucagon.

In one embodiment the glucagon RA is chosen in the group of human glucagon and dasiglucagon (Dasiglucagon^{®}, Zealand Pharma).

Human glucagon is a peptide having the sequence: HSQGTFTSDY SKYLDSRRAQ DFVQWLMNT (SEQ ID n°4)

Dasiglucagon is a peptide having the sequence: HSQGTFTSDY SKYLDXARAE EFVKWLEST where X is Aib (SEQ ID n°5)

In one embodiment the glucagon RA is human glucagon.

In one embodiment the glucagon RA is dasiglucagon.

In one embodiment the concentration in glucagon RA is comprised from 10 to 15000 µg/mL.

In one embodiment the concentration in glucagon RA is comprised from 100 to 10000 µg/mL.

In one embodiment the concentration in glucagon RA is comprised from 1000 to 5000 µg/mL.

In one embodiment the human glucagon concentration is comprised from 30 to 12000 µg/mL.

In one embodiment the human glucagon concentration is comprised from 100 to 12000 µg/mL.

In one embodiment the human glucagon concentration is comprised from 1000 to 10000 µg/mL.

In one embodiment the human glucagon concentration is comprised from 1000 to 5000 µg/mL.

In one embodiment the human glucagon concentration is comprised from 2000 to 4000 µg/mL.

In one embodiment the dasiglucagon concentration is comprised from 10 to 5000 µg/mL.

In one embodiment the dasiglucagon concentration is comprised from 100 to 4000 µg/mL.

In one embodiment the dasiglucagon concentration is comprised from 200 to 3000 µg/mL.

In one embodiment the dasiglucagon concentration is comprised from 200 to 2000 µg/mL.

In one embodiment the dasiglucagon concentration is comprised from 300 to 1500 µg/mL.

In an embodiment, the composition allows the delivery of a daily dose of human glucagon ranging from 250 to 10000 µg/day.

In an embodiment, the composition allows the delivery of a daily dose of human glucagon ranging from 500 to 7500 µg/day.

In an embodiment, the composition allows the delivery of a daily dose of human glucagon ranging from 500 to 5000 µg/day.

In an embodiment, the composition allows the delivery of a daily dose of dasiglucagon ranging from 100 to 5000 µg/day.

In an embodiment, the composition allows the delivery of a daily dose of dasiglucagon ranging from 250 to 3000 µg/day.

In an embodiment, the composition allows the delivery of a daily dose of dasiglucagon ranging from 250 to 2000 µg/day.

In an embodiment, the composition comprises a molar ratio between glucagon RA and GLP-1 RA ranging from 1 to 60.

In an embodiment the molar ratio between glucagon RA and GLP-1 RA in the composition is ranging from 2 to 50.

In an embodiment the molar ratio between glucagon RA and GLP-1 RA in the composition is ranging from 2 to 25.

In an embodiment the molar ratio between human glucagon and exenatide in the composition is ranging from 3 to 35.

In an embodiment the molar ratio between human glucagon and exenatide in the composition is ranging from 4 to 25.

In an embodiment the molar ratio between human glucagon and exenatide in the composition is ranging from 5 to 20.

In an embodiment the molar ratio between human glucagon and exenatide in the composition is ranging from 8 to 60.

In an embodiment the molar ratio between human glucagon and exenatide in the composition is ranging from 10 to 55.

In an embodiment the molar ratio between human glucagon and exenatide in the composition is ranging from 15 to 40.

In an embodiment the molar ratio between human glucagon and lixisenatide in the composition is ranging from 2 to 40.

In an embodiment the molar ratio between human glucagon and lixisenatide in the composition is ranging from 3 to 30.

In an embodiment the molar ratio between human glucagon and lixisenatide in the composition is ranging from 4 to 20.

In an embodiment the molar ratio between dasiglucagon and exenatide in the composition is ranging from 0.5 to 15.

In an embodiment the molar ratio between dasiglucagon and exenatide in the composition is ranging from 1 to 10.

In an embodiment the molar ratio between dasiglucagon and exenatide in the composition is ranging from 1.2 to 8.

In an embodiment the molar ratio between dasiglucagon and exenatide in the composition is ranging from 1 to 40.

In an embodiment the molar ratio between dasiglucagon and exenatide in the composition is ranging from 2 to 25.

In an embodiment the molar ratio between dasiglucagon and exenatide in the composition is ranging from 3 to 16.

In an embodiment the molar ratio between dasiglucagon and lixisenatide in the composition is ranging from 0.3 to 15.

In an embodiment the molar ratio between dasiglucagon and lixisenatide in the composition is ranging from 0.5 to 10.

In an embodiment the molar ratio between dasiglucagon and lixisenatide in the composition is ranging from 1 to 7.

In an embodiment, the administration of the composition leads, at steady state, to a molar ratio of exenatide and human glucagon ranging from 0.3 to 15.

In an embodiment, the administration of the composition leads, at steady state, to a molar ratio of exenatide and human glucagon ranging from 0.5 to 8.

In an embodiment, the administration of the composition leads, at steady state, to a molar ratio of exenatide and human glucagon ranging from 0.8 to 4.

In an embodiment, the administration of the composition leads, at steady state, to a molar ratio of exenatide and dasiglucagon ranging from 0.3 to 15.

In an embodiment, the administration of the composition leads, at steady state, to a molar ratio of exenatide and dasiglucagon ranging from 0.5 to 8.

In an embodiment, the administration of the composition leads, at steady state, to a molar ratio of exenatide and dasiglucagon ranging from 0.8 to 4.

In an embodiment, the administration of the composition leads, at steady state, to a molar ratio of lixisenatide and human glucagon ranging from 0.1 to 2.

In an embodiment, the administration of the composition leads, at steady state, to a molar ratio of lixisenatide and human glucagon ranging from 0.25 to 1.5.

In an embodiment, the administration of the composition leads, at steady state, to a molar ratio of lixisenatide and human glucagon ranging from 0.3 to 1.3.

In an embodiment, the administration of the composition leads, at steady state, to a molar ratio of lixisenatide and dasiglucagon ranging from 0.1 to 2.

In an embodiment, the administration of the composition leads, at steady state, to a molar ratio of lixisenatide and dasiglucagon ranging from 0.25 to 1.5.

In an embodiment, the administration of the composition leads, at steady state, to a molar ratio of lixisenatide and dasiglucagon ranging from 0.3 to 1.3.

In an embodiment, the composition is a clear solution.

In another embodiment the composition is an injectable solution.

According to an embodiment, the composition is an aqueous injectable solution.

According to an embodiment, the composition is an injectable solution comprising an aprotic polar solvent.

According to an embodiment the composition comprises at least 10 % w/w of water.

According to an embodiment the composition comprises at least 50 % w/w of water.

According to an embodiment the composition comprises at least 70 % w/w of water.

According to an embodiment the composition comprises at least 80 % w/w of water.

According to an embodiment the composition comprises at least 10 % of water.

According to an embodiment the composition is a solution comprising at least 10 % v/v of water.

According to an embodiment the composition is a solution comprising at least 30 % v/v of water.

According to an embodiment the composition is a solution comprising at least 50 % v/v of water.

According to an embodiment the composition is a solution comprising at least 70 % v/v of water.

According to an embodiment the composition is a solution comprising at least 75 % v/v of water.

According to an embodiment the composition is a solution comprising at least 80 % v/v of water.

According to an embodiment the composition is a solution comprising at least 85 % v/v of water.

According to an embodiment the composition is an aqueous injectable solution. By "aqueous solution" is meant that at least 80 % of the volume of the solution is water.

According to an embodiment, the aqueous solution comprises less than 1 % v/v of aprotic polar solvent.

According to an embodiment, the composition comprises an aprotic polar solvent.

In an embodiment, the aprotic polar solvent is chosen from the group consisting of dimethylsulfoxide (DMSO), *N,N*-dimethylformamide (DMF), ethyl acetate, *N*-methyl-2-pyrrolidone (NMP), *N,N*-dimethylacetamide (DMA), and propylene carbonate.

In an embodiment, the aprotic polar solvent is DMSO, NMP or a mixture thereof.

In an embodiment, the aprotic polar solvent is DMSO.

According to an embodiment, the composition comprises at least 10 % of an aprotic polar solvent.

According to an embodiment the composition is a solution comprising at least 10 % v/v of aprotic polar solvent.

According to an embodiment the composition is a solution comprising at least 20 % v/v of aprotic polar solvent.

According to an embodiment the composition is a solution comprising at least 30 % v/v of aprotic polar solvent.

According to an embodiment the composition is a solution comprising at least 40 % v/v of aprotic polar solvent.

According to an embodiment the composition is a solution comprising at least 50 % v/v of aprotic polar solvent.

According to an embodiment the composition is a solution comprising at least 60 % v/v of aprotic polar solvent.

According to an embodiment the composition is a solution comprising at least 70 % v/v of aprotic polar solvent.

According to an embodiment the composition is a solution comprising at least 80 % v/v of aprotic polar solvent.

By "aprotic polar solvent solution" is meant a solution comprising at least 50 % v/v of aprotic polar solvent.

According to an embodiment the composition is a solution comprising an aprotic polar solvent and at least 10 % v/v of water.

According to an embodiment the composition is a solution comprising an aprotic polar solvent and at least 20 % v/v of water.

According to an embodiment the composition is a solution comprising an aprotic polar solvent and at least 30 % v/v of water.

According to an embodiment the composition is a solution comprising an aprotic polar solvent and at least 40 % v/v of water.

According to an embodiment the composition is a solution comprising an aprotic polar solvent and at least 50 % v/v of water.

According to an embodiment the composition is a solution comprising from 50 to 90 % v/v of aprotic polar solvent and from 10 to 50 % v/v of water.

According to an embodiment, the composition comprising an aprotic polar solvent further comprises 1 to 50 mM of an ionization stabilizing excipient.

According to an embodiment, the composition comprising an aprotic polar solvent further comprises 2 to 20 mM of an ionization stabilizing excipient.

According to an embodiment, the composition comprising an aprotic polar solvent further comprises 2 to 10 mM of an ionization stabilizing excipient.

According to an embodiment, the composition comprising an aprotic polar solvent further comprises 3 to 7 mM of an ionization stabilizing excipient.

According to an embodiment the ionization stabilizing excipient is a mineral acid. Said mineral acid may be selected from hydrochloric acid, sulfuric acid, nitric acid and phosphoric acid.

According to an embodiment the ionization stabilizing excipient is an organic acid, such as acids having a carboxylic acid -COOH functional group. Said organic acids may be selected from acetic acid, citric acid, and amino acids.

When the composition is a solution, the concentrations are expressed in weight or Units to volume, such as U/mL, mg/ml or µg/mL.

When the composition is in liquid form that contains enough water to allow a reliable measure of pH, its pH may be as listed below.

In an embodiment the pH of the composition is ranging from 3.0 to 8.0.

In an embodiment the pH of the composition is ranging from 3.2 to 7.8.

In an embodiment the pH of the composition is ranging from 3.4 to 7.6.

In an embodiment the pH of the composition is ranging from 3.6 to 7.4.

In an embodiment the pH of the composition is ranging from 3.4 to 7.2.

In an embodiment the pH of the composition is ranging from 3.2 to 7.0.

In an embodiment the pH of the composition is ranging from 3.0 to 6.8.

In an embodiment the pH of the composition is ranging from 3.0 to 6.6.

In an embodiment the pH of the composition is ranging from 3.0 to 4.4.

In an embodiment the pH of the composition is ranging from 3.2 to 4.8.

In an embodiment the pH of the composition is ranging from 3.5 to 4.4.

In an embodiment the pH of the composition is ranging from 3.6 to 4.4.

In an embodiment the pH of the composition is ranging from 3.7 to 4.3.

In an embodiment the pH of the composition is ranging from 3.5 to 4.2.

In an embodiment the pH of the composition is ranging from 3.6 to 4.4.

In an embodiment the pH of the composition is ranging from 3.7 to 4.0.

In another embodiment the pH of the composition is ranging from 3.8 to 4.2.

In another embodiment the pH of the composition is ranging from 3.2 to 3.8.

In another embodiment the pH of the composition is ranging from 3.3 to 3.7.

In another embodiment the pH of the composition is 4.0.

In another embodiment the pH of the composition is 3.9.

In another embodiment the pH of the composition is 3.8.

In another embodiment the pH of the composition is 3.7.

In another embodiment the pH of the composition is 3.6.

In an embodiment the pH of the composition is 3.5.

In an embodiment the pH of the composition is 3.4.

In an embodiment the pH of the composition is ranging from 5.0 to 8.0.

In an embodiment the pH of the composition is ranging from 5.5 to 7.8,

In an embodiment the pH of the composition is ranging from 6.0 to 7.6.

In an embodiment the pH of the composition is ranging from 6.5 to 7.4.

In an embodiment the pH of the composition is ranging from 6.8 to 7.4.

According to an embodiment the composition comprises one short-acting GLP-1 RA and one short-acting amylin RA.

According to an embodiment the composition comprises exenatide and pramlintide.

According to an embodiment the composition comprises lixisenatide and pramlintide.

According to an embodiment the composition comprises one short-acting GLP-1 RA and one short-acting glucagon RA.

According to an embodiment the composition comprises exenatide and human glucagon.

According to an embodiment the composition comprises exenatide and dasiglucagon.

According to an embodiment the composition comprises lixisenatide and human glucagon.

According to an embodiment the composition comprises lixisenatide and dasiglucagon.

According to an embodiment the composition comprises one short-acting glucagon RA and one short-acting amylin RA.

According to an embodiment the composition comprises human glucagon and pramlintide.

According to an embodiment the composition comprises dasiglucagon and pramlintide.

According to an embodiment the composition comprises one short-acting glucagon RA, one short-acting GLP-1 RA and one short-acting amylin RA.

According to an embodiment the composition comprises pramlintide, human glucagon and exenatide.

According to an embodiment the composition comprises pramlintide, human glucagon and lixisenatide.

According to an embodiment the composition comprises pramlintide, dasiglucagon and exenatide.

According to an embodiment the composition comprises pramlintide, dasiglucagon and lixisenatide.

According to an embodiment the composition comprises at least 2 short-acting peptides chosen in at least two of the following groups, GLP-1 RA, Amylin RA and Glucagon RA and a copolyamino acid bearing carboxylate charges and hydrophobic radicals Hy.

According to an embodiment the compositions comprises a copolyamino acid bearing carboxylate charges and hydrophobic radicals Hy , said copolyamino acid consisting of glutamic or aspartic units and said hydrophobic radicals -Hy being chosen among the radicals according to formula X as defined below: 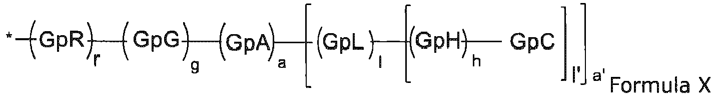 wherein
- GpR is chosen among the radicals according to formulas VII, VII' or VII": 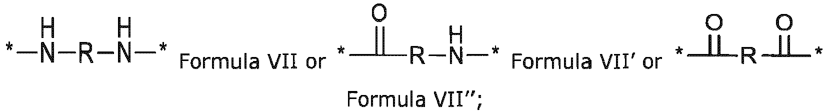
- GpG and GpH identical or different are chosen among the radicals according to formulas XI or XI': 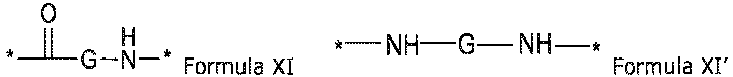
- GpA is chosen among the radicals according to formula VIIIa or VIIIb 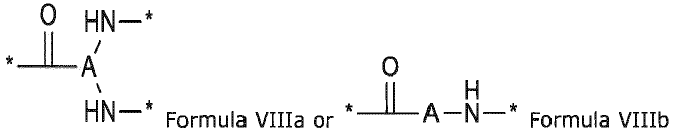
- -GpL is a radical according to formula XII 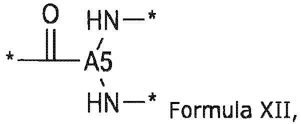
- GpC is a radical according to formula IX: 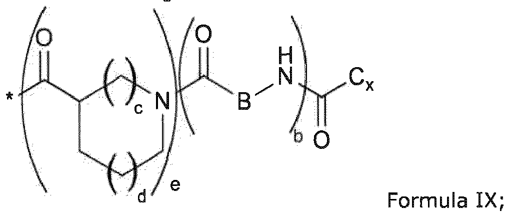
- the ^{∗} indicate the binding sites of the different groups bound by an amide function;
- a is an integer equal to 0 or to 1 and a' = 1 if a = 0 and a' = 1, 2 or 3 if a = 1;
- a' is an integer equal to 1, to 2 or to 3;
- b is an integer equal to 0 or to 1;
- c is an integer equal to 0 or to 1, and if c is equal to 0 then d is equal to 1 or to 2;
- d is an integer equal to 0, to 1 or to 2;
- e is an integer equal to 0 or to 1;
- g is an integer equal to 0, to 1, to 2, to 3 to 4 to 5 or to 6;
- h is an integer equal to 0, to 1, to 2, to 3 to 4 to 5 or to 6;
- I is an integer equal to 0 or 1 and I' = 1 if I = 0 and I' = 2 if I = 1;
- I' is an integer equal to 1 or to 2;
- r is an integer equal to 0, 1 or to 2, and
- s' is an integer equal to 0 or 1;
- And if e is different from 0, then at least one of g, h or I is different from 0
- And if a = 0, then I = 0;
- A and As identical or different are linear or branched alkyl radicals comprising from 1 to 8 carbon atoms, and optionally substituted by a radical from a saturated, unsaturated or aromatic ring;
- B is a radical chosen in the group consisting of a non-substituted ether or polyether radical comprising from 4 to 14 carbon atoms and from 1 to 5 oxygen atoms or a linear or branched alkyl radical, optionally comprising an aromatic nucleus, comprising from 1 to 9 carbon atoms;
- Cₓ is a radical chosen in the group consisting of a linear or branched monovalent alkyl radical, optionally comprising a cyclic part, wherein x indicates the number of carbon atoms and 6≤x≤25, in particular
   - When the hydrophobic radical -Hy bears 1 -GpC, then 9 ≤ x ≤ 25,
   - When the hydrophobic radical -Hy bears 2 -GpC, then 9 ≤ x ≤ 15,
- G is a linear or branched divalent alkyl radical of 1 to 8 carbon atoms, said alkyl radical bearing one or a plurality of free carboxylic acid function(s),
- R is a radical chosen in the group consisting of a linear or branched, divalent alkyl radical comprising from 1 to 12 carbon atoms or a non-substituted ether or polyether radical comprising from 4 to 14 carbon atoms and from 1 to 5 oxygen atoms.

In an embodiment, e = 1.

In an embodiment said hydrophobic radicals are chosen amongst the radicals according to formula X wherein g=I=0 according to formula Xa as defined below :
-(GpR)r-(GpA)a-[(GpH)h-GpC]I' formula Xa wherein GpR, GpA, GpH, GpC, r, a, h and I' are as defined according to formula X.

In one embodiment, said hydrophobic radicals -Hy is chosen among radicals according to formula X wherein g=I=0 according to formula Xa as defined below :
-(GpR)ᵣ-(GpA)ₐ-[(GpH)ₕ-GpC]_{l'} formula Xa wherein GpC is a radical according to formula IX wherein e=1 and b=0 and GpC is according to the following formula IXa:

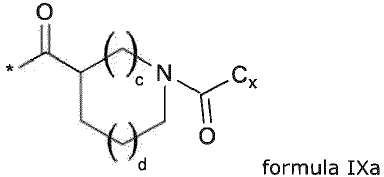

In one embodiment, said hydrophobic radical -Hy is chosen radicals according to formula X wherein g=I=0 according to formula Xa as defined below :
-(GpR)ᵣ-(GpA)ₐ-[(GpH)ₕ-GpC]_{l'} formula Xa wherein GpR, GpA, GpH, GpC, r, h, a and I' are as defined above and h = 1, 2 or 3.

In one embodiment, said hydrophobic radical -Hy is chosen radicals according to formula X wherein g=I=0 and a=1 and l'=2 according to formula Xb as defined below :
-(GpR)ᵣ-GpA-[(GpH)ₕ-GpC]₂ formula Xb wherein GpR, GpA, GpH, GpC, h and r are as defined above.

In one embodiment, said hydrophobic radical -Hy is chosen radicals according to formula X wherein g=l=0 and a=1 and l'=2 according to formula Xb as defined below :
-(GpR)ᵣ-GpA-[(GpH)ₕ-GpC]₂ formula Xb wherein GpR, GpA, GpH, r and h are as defined above and GpC is according to formula IXa as defined below :

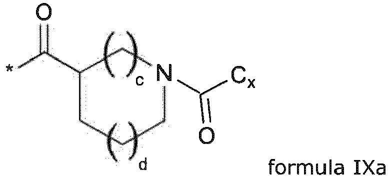

In one embodiment, said hydrophobic radical -Hy is chosen radicals according to formula X wherein g=I=0 and a=1 and l'=2 according to formula Xb as defined below :
-(GpR)ᵣ-GpA-[(GpH)ₕ-GpC]₂ formula Xb wherein GpR, GpA, GpH, GpC and r are as defined above and h = 1, 2 or 3.

In an embodiment said hydrophobic radicals are chosen among the radicals according to formula X wherein g=I=0 and r= 1 according to formula Xc as defined below:
-GpR- (GpA)a-[(GpH)h-GpC]l' formula Xc wherein GpR is a radical according to formula VII 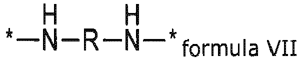 And GpA, GpH, GpC, al, h and I' are as defined above.

In an embodiment said hydrophobic radicals are chosen amongst the radicals according to formula X wherein r=g=I=0 according tot formula Xd as defined below:
-(GpA)ₐ-[(GpH)ₕ-GpC]_{l'} formula Xd wherein GpA, GpH, GpC, a, h and I' are as defined above.

In an embodiment, said hydrophobic radical is according to formula X, Xa , Xb, Xc or Xd in which h = 1, 2 or 3.

In an embodiment, said hydrophobic radical is according to formula X, Xa Xb, Xc or Xd in which h = 1.

In an embodiment, said hydrophobic radical is according to formula X, Xa Xb, Xc or Xd in which h = 2.

In an embodiment, said hydrophobic radical is according to formula X, Xa Xb, Xc or Xd in which h = 3.

In an embodiment, the composition is characterized in that the hydrophobic radical according to formula X, Xa, Xb, Xc or Xd is a radical wherein R is a divalent linear alkyl radical comprising from 2 to 12 carbon atoms.

In an embodiment, the composition is characterized in that the hydrophobic radical according to formula X, Xa, Xb, Xc or Xd is a radical wherein R is a divalent linear alkyl radical comprising from 2 to 6 carbon atoms.

In an embodiment, the composition is characterized in that the hydrophobic radical according to formula X, Xa, Xb, Xc or Xd is a radical wherein R is a divalent linear alkyl radical comprising from 2 to 4 carbon atoms.

In an embodiment, the composition is characterized in that the hydrophobic radical according to formula X, Xa, Xb, Xc or Xd is a radical wherein R is a divalent linear alkyl radical comprising 2 carbon atoms.

In an embodiment, the composition is characterized in that the hydrophobic radical according to formula X, Xa, Xb, Xc or Xd is a radical wherein R is a divalent branched alkyl radical comprising from 2 to 12 atoms of carbon.

In an embodiment, the composition is characterized in that the hydrophobic radical according to formula X, Xa, Xb, Xc or Xd is a radical wherein R is an unsubstituted linear ether or polyether radical comprising 4 to 14 carbon atoms and from 1 to 5 oxygen atoms.

In an embodiment, the composition is characterized in that the hydrophobic radical according to formula X, Xa, Xb, Xc or Xd is a radical wherein R is an ether radical.

In an embodiment, the composition according to the invention is characterized in that the hydrophobic radical according to formula X, Xa, Xb, Xc or Xd wherein the radical GpA is according to formula VIIIa or VIIIb and in which A is chosen from the group consisting of the radicals represented by the formulas below:

| | | |
|---|---|---|
| 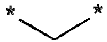 | 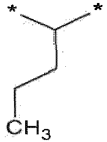 | 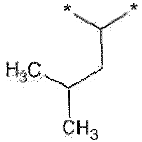 |
| Formula Y1 | Formula Y2 | Formula Y3 |
| 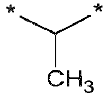 | 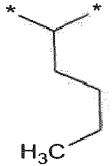 | 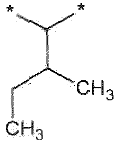 |
| Formula Y4 | Formula Y5 | Formula Y6 |
| 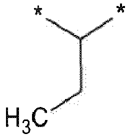 | 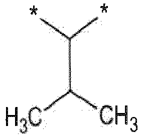 | 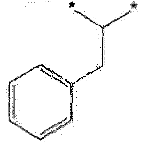 |
| Formula Y7 | Formula Y8 | Formula Y9 |
| 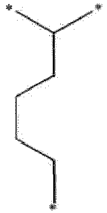 | | |
| Formula Y10 | | |

In one embodiment, the composition is characterized in that the hydrophobic radical according to formulas X, Xa, Xb, Xc or Xd is a radical wherein the radical GpG and/or GpH is according to formula XI' in which G is an alkyl radical comprising 6 carbon atoms represented by formula Z below:

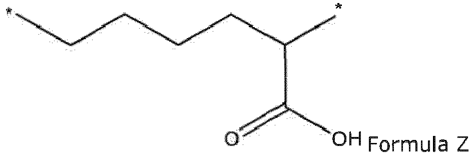

In one embodiment, the composition is characterized in that the hydrophobic radical according to formulas X, Xa, Xb, Xc or Xd is a radical wherein the radical GpG and/or GpH is according to formula XI wherein is an alkyl radical comprising 4 carbon atoms represented by formula Z below:

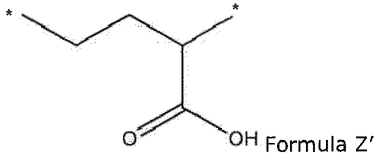

In one embodiment, the composition is characterized in that the hydrophobic radical according to formulas X, Xa, Xb, Xc or Xd is a radical wherein the radical GpC according to formula IXa, is chosen among radicals according to formulas IXb, IXc, and IXd represented below:

| | |
|---|---|
| 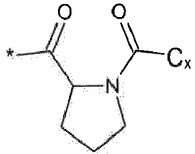 | Formula IXb |
| 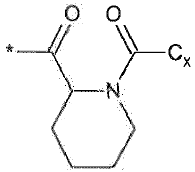 | Formula IXc |
| 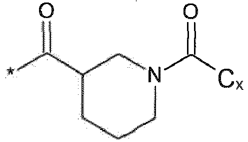 | Formula IXd |

In one embodiment, the composition is characterized in that the hydrophobic radical according to formulas X, Xa, Xb, Xc or Xd is a radical wherein Cx is a linear or branched monovalent alkyl radical comprising from 9 to 25 carbon atoms.

In one embodiment, the composition is characterized in that the hydrophobic radical according to formulas X, Xa, Xb, Xc or Xd is a radical wherein Cx is a linear or branched monovalent alkyl radical comprising from 11 to 17 carbon atoms.

In one embodiment, the composition is characterized in that the hydrophobic radical according to formulas X, Xa, Xb, Xc or Xd is a radical wherein Cx is a linear or branched monovalent alkyl radical comprising from 13 to 15 carbon atoms

In one embodiment, the composition is characterized in that the hydrophobic radical according to formulas X, Xa, Xb, Xc or Xd is a radical wherein Cx is a linear or branched monovalent alkyl radical comprising 13 carbon atoms.

In one embodiment, the composition is characterized in that the hydrophobic radical according to formulas X, Xa, Xb, Xc or Xd is a radical wherein Cx is a linear or branched monovalent alkyl radical comprising 15 carbon atoms.

In one embodiment, the composition according to the invention is characterized in that the copolyamino acid bearing carboxylate charges and hydrophobic radicals is chosen from the copolyamino acids according to the following formula XXXa 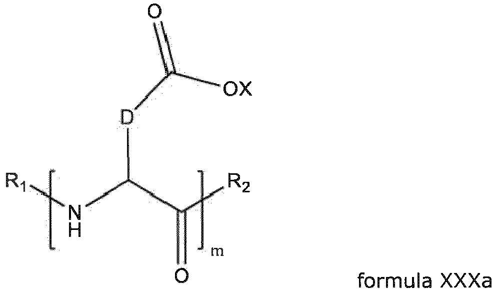 wherein
- D represents, independently, either a -CH₂- group (aspartic unit) or a -CH₂-CH₂-group (glutamic unit),
- Hy is a hydrophobic radical chosen from the hydrophobic radicals according to formula X,
- R₁ is a hydrophobic radical chosen from the hydrophobic radicals according to formula X or a radical chosen from the group consisting of an H, a linear acyl group in C2 to C10, a branched acyl group in C4 to C10, a benzyl, a terminal amino acid unit and a pyroglutamate,
- R₂ is a hydrophobic radical chosen from the hydrophobic radicals according to formula X in which r=1 and GpR is a radical according to formula VII, or a radical - NR'R", with R' and R", either identical or different, being chosen from the group comprised by H, the linear or branched or cyclic alkyls in C2 to C10, the benzyl and said alkyls R' and R" which may form together one or more saturated, unsaturated and/or aromatic carbonated cycles, and/or may comprise hetero-atoms, chosen from the group comprised of O, N and S;
- at least R₁ or R₂ is a hydrophobic radical according to formula X,
- X represents a cationic entity chosen from the group comprising alkaline cations;
- m represents the degree of polymerization DP of the copolyamino acid, that means, the average number of monomeric units per copolyamino acid chain and 5 ≤ m ≤ 250.

In one embodiment, the composition according to the invention is characterized in that the copolyamino acid bearing carboxylate charges and hydrophobic radicals is chosen among the copolyamino acids according to formula XXXa and R₁ is a hydrophobic radical according to formula X.

In one embodiment, the composition according to the invention is characterized in that the copolyamino acid bearing carboxylate charges and hydrophobic radicals is chosen among the copolyamino acids according to formula XXXa and R₂ is a hydrophobic radical according to formula X.

In one embodiment, the composition according to the invention is characterized in that the copolyamino acid bearing carboxylate charges and hydrophobic radicals is chosen from the copolyamino acids according to the following formula XXXb 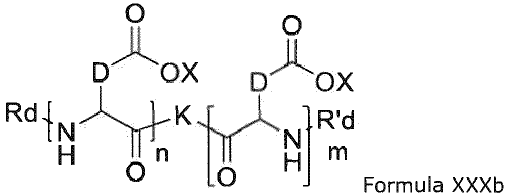 wherein
- D represents, independently, either a -CH₂- group (aspartic unit) or a -CH₂-CH₂-group (glutamic unit),
- X represents a cationic entity chosen from the group comprising alkaline cations;
- Rd et R'd, identical or different, are a hydrophobic radical -Hy according to formula X,
- K is a divalent linker chosen among the following formulas III or IV, 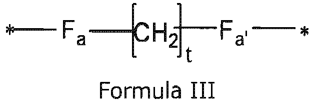
wherein
∘ 1 ≤ t ≤ 8, this means that t is an integer comprised from 1 to 8,
∘ Fa et Fa', identical or different represent a -NH- function 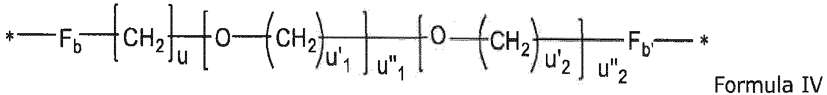
wherein :
- Fb et Fb', identical or different represent a -NH- function
- at least one of u₁" or u₂" is different from 0.
- if u₁" ≠ 0 then u₁' ≠ 0 and if u₂" ≠ 0 then u₂' ≠ 0,
- u₁' and u₂' are identical or different and,
- 2 ≤ u ≤ 4,
- 0 ≤ u₁' ≤ 4,
- 0 ≤ u₁" ≤ 4,
- 0 ≤ u₂' ≤ 4,
- 0 ≤ u2" ≤ 4,
- said linker K is bound to the at least two glutamic or aspartic chains of the copolyamino acid by amides functions;
- n + m represents the degree of polymerization DP of the copolyamino acid, that means, the average number of monomeric units per copolyamino acid chain and 5 ≤ n + m ≤ 250.

In one embodiment, the composition according to the invention is characterized in that the copolyamino acid bearing carboxylate charges and hydrophobic radicals is chosen from the copolyamino acids according to the following formula XXXc 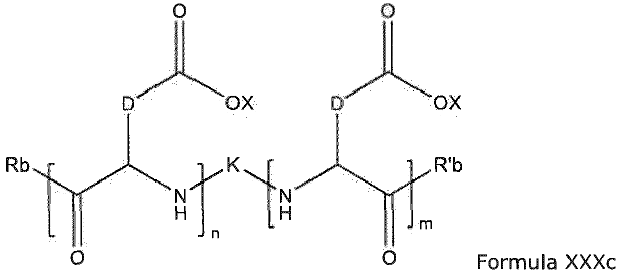 wherein,
- D and X are as defined according to formula XXXb,
- Rb and R'b, identical or different, are a hydrophobic radical -Hy,
- K is as defined according to formula XXXb, except than

∘ Fa and Fa', identical or different represent a -CO- function and
∘ Fb and Fb', identical or different represent a -CO- function.

- n + m is as defined according to formula XXXb.

In an embodiment the composition comprises the copolyamino acid bearing carboxylate charges and hydrophobic radicals in a concentration ranging from 1 to 50 mg/ml.

In an embodiment the composition comprises the copolyamino acid bearing carboxylate charges and hydrophobic radicals in a concentration ranging from 2 to 30 mg/ml.

In an embodiment the composition comprises the copolyamino acid bearing carboxylate charges and hydrophobic radicals in a concentration ranging from 4 to 20 mg/ml.

In an embodiment the composition comprises the copolyamino acid bearing carboxylate charges and hydrophobic radicals in a concentration ranging from 5 to 15 mg/ml.

According to an embodiment the composition is an aprotic polar solvent injectable solution comprising one short-acting GLP-1 RA and one short-acting amylin RA.

According to an embodiment the composition is an aprotic polar solvent injectable solution comprising exenatide and pramlintide.

According to an embodiment the composition is an aprotic polar solvent injectable solution comprising lixisenatide and pramlintide.

According to an embodiment the composition is an aprotic polar solvent injectable solution comprising one short-acting GLP-1 RA and one short-acting glucagon RA.

According to an embodiment the composition is an aprotic polar solvent injectable solution comprising exenatide and human glucagon.

According to an embodiment the composition is an aprotic polar solvent injectable solution comprising exenatide and dasiglucagon.

According to an embodiment the composition is an aprotic polar solvent injectable solution comprising lixisenatide and human glucagon.

According to an embodiment the composition is an aprotic polar solvent injectable solution comprising lixisenatide and dasiglucagon.

According to an embodiment the composition is an aprotic polar solvent injectable solution comprising one short-acting glucagon RA and one short-acting amylin RA.

According to an embodiment the composition is an aprotic polar solvent injectable solution comprising human glucagon and pramlintide.

According to an embodiment the composition is an aprotic polar solvent injectable solution comprising dasiglucagon and pramlintide.

According to an embodiment the composition is an aprotic polar solvent injectable solution comprising one short-acting glucagon RA, one short-acting GLP-1 RA and one short-acting amylin RA.

According to an embodiment the composition is an aprotic polar solvent injectable solution comprising pramlintide, human glucagon and exenatide.

According to an embodiment the composition is an aprotic polar solvent injectable solution comprising pramlintide, human glucagon and lixisenatide.

According to an embodiment the composition is an aprotic polar solvent injectable solution comprising pramlintide, dasiglucagon and exenatide.

According to an embodiment the composition is an aprotic polar solvent injectable solution comprising pramlintide, dasiglucagon and lixisenatide.

According to an embodiment the composition is an aqueous solution having a pH ranging from 3.0 to 8.0.

According to an embodiment the composition is an aqueous solution having a pH ranging from 3.6 to 7.6.

According to an embodiment the composition is an aqueous solution having a pH ranging from 3.0 to 6.6.

According to an embodiment the composition is an aqueous injectable solution having a pH ranging from 3.0 to 8.0, in particular ranging from 3.6 to 7.6 and more particularly from 3.0 to 6.6, and comprising one short-acting GLP-1 RA and one short-acting amylin RA.

According to an embodiment the composition is an aqueous injectable solution having a pH ranging from 3.0 to 8.0, in particular ranging from 3.6 to 7.6 and more particularly from 3.0 to 6.6, and comprising exenatide and pramlintide.

According to an embodiment the composition is an aqueous injectable solution having a pH ranging from 3.0 to 8.0, in particular ranging from 3.6 to 7.6 and more particularly from 3.0 to 6.6, and comprising lixisenatide and pramlintide.

According to an embodiment the composition is an aqueous injectable solution having a pH ranging from 3.0 to 8.0, in particular ranging from 3.6 to 7.6 and more particularly from 3.0 to 6.6, and comprising one short-acting GLP-1 RA and one short-acting glucagon RA.

According to an embodiment the composition is an aqueous injectable solution having a pH ranging from 3.0 to 8.0, in particular ranging from 3.6 to 7.6 and more particularly from 3.0 to 6.6, and comprising exenatide and dasiglucagon.

According to an embodiment the composition is an aqueous injectable solution having a pH ranging from 3.0 to 8.0, in particular ranging from 3.6 to 7.6 and more particularly from 3.0 to 6.6, and comprising lixisenatide and dasiglucagon.

According to an embodiment the composition is an aqueous injectable solution having a pH ranging from 3.0 to 8.0, in particular ranging from 3.6 to 7.6 and more particularly from 3.0 to 6.6, and comprising one short-acting glucagon RA and one short-acting amylin RA.

According to an embodiment the composition is an aqueous injectable solution having a pH ranging from 3.0 to 8.0, in particular ranging from 3.6 to 7.6 and more particularly from 3.0 to 6.6, and comprising dasiglucagon and pramlintide.

According to an embodiment the composition is an aqueous injectable solution having a pH ranging from 3.0 to 8.0, in particular ranging from 3.6 to 7.6 and more particularly from 3.0 to 6.6, and comprising one short-acting glucagon RA, one short-acting GLP-1 RA and one short-acting amylin RA.

According to an embodiment the composition is an aqueous injectable solution having a pH ranging from 3.0 to 8.0, in particular ranging from 3.6 to 7.6 and more particularly from 3.0 to 6.6, and comprising pramlintide, dasiglucagon and exenatide.

According to an embodiment the composition is an aqueous injectable solution having a pH ranging from 3.0 to 8.0, in particular ranging from 3.6 to 7.6 and more particularly from 3.0 to 6.6, and comprising pramlintide, dasiglucagon and lixisenatide.

According to an embodiment the composition is an aqueous solution having a pH ranging from 3.0 to 4.8.

According to an embodiment the composition is an aqueous solution having a pH ranging from 3.3 to 4.4.

According to an embodiment the composition is an aqueous injectable solution having a pH ranging from 3.0 to 4.8, in particular ranging from 3.3 to 4.4, and comprising one short-acting GLP-1 RA and one short-acting amylin RA.

According to an embodiment the composition is an aqueous injectable solution having a pH ranging from 3.0 to 4.8, in particular ranging from 3.3 to 4.4, and comprising exenatide and pramlintide.

According to an embodiment the composition is an aqueous injectable solution having a pH ranging from 3.0 to 4.8, in particular ranging from 3.3 to 4.4, and comprising lixisenatide and pramlintide.

According to an embodiment the composition is an aqueous injectable solution having a pH ranging from 3.0 to 4.8, in particular ranging from 3.3 to 4.4, and comprising one short-acting GLP-1 RA and one short-acting glucagon RA.

According to an embodiment the composition is an aqueous injectable solution having a pH ranging from 3.0 to 4.8, in particular ranging from 3.3 to 4.4, and comprising exenatide and dasiglucagon.

According to an embodiment the composition is an aqueous injectable solution having a pH ranging from 3.0 to 4.8, in particular ranging from 3.3 to 4.4, and comprising lixisenatide and dasiglucagon.

According to an embodiment the composition is an aqueous injectable solution having a pH ranging from 3.0 to 4.8, in particular ranging from 3.3 to 4.4, and comprising one short-acting glucagon RA and one short-acting amylin RA.

According to an embodiment the composition is an aqueous injectable solution having a pH ranging from 3.0 to 4.8, in particular ranging from 3.3 to 4.4, and comprising dasiglucagon and pramlintide.

According to an embodiment the composition is an aqueous injectable solution having a pH ranging from 3.0 to 4.8, in particular ranging from 3.3 to 4.4, and comprising one short-acting glucagon RA, one short-acting GLP-1 RA and one short-acting amylin RA.

According to an embodiment the composition is an aqueous injectable solution having a pH ranging from 3.0 to 4.8, in particular ranging from 3.3 to 4.4, and comprising pramlintide, dasiglucagon and exenatide.

According to an embodiment the composition is an aqueous injectable solution having a pH ranging from 3.0 to 4.8, in particular ranging from 3.3 to 4.4, and comprising pramlintide, dasiglucagon and lixisenatide.

According to an embodiment the composition is an aqueous solution having a pH ranging from 5.0 to 8.0.

According to an embodiment the composition is an aqueous solution having a pH ranging from 5.5 to 7.8.

According to an embodiment the composition is an aqueous injectable solution having a pH ranging from 5.0 to 8.0, in particular ranging from 5.5 to 7.8, and comprising one short-acting GLP-1 RA and one short-acting amylin RA.

According to an embodiment the composition is an aqueous injectable solution having a pH ranging from 5.0 to 8.0, in particular ranging from 5.5 to 7.8, and comprising one short-acting GLP-1 RA and one short-acting glucagon RA.

According to an embodiment the composition is an aqueous injectable solution having a pH ranging from 5.0 to 8.0, in particular ranging from 5.5 to 7.8, and comprising exenatide and dasiglucagon.

According to an embodiment the composition is an aqueous injectable solution having a pH ranging from 5.0 to 8.0, in particular ranging from 5.5 to 7.8, and comprising one short-acting glucagon RA and one short-acting amylin RA.

According to an embodiment the composition is an aqueous injectable solution having a pH ranging from 5.0 to 8.0, in particular ranging from 5.5 to 7.8, and comprising one short-acting glucagon RA, one short-acting GLP-1 RA and one short-acting amylin RA.

According to an embodiment the composition is an aqueous solution having a pH ranging from 5.0 to 8.0, in particular from 6.0 to 7.8, comprising a copolyamino acid bearing carboxylate charges and hydrophobic radicals according to formula X and at least two short-acting peptides chosen in at least two of the following groups, GLP-1 RA, amylin RA and glucagon RA.

In an embodiment the composition does not comprise long-acting peptides.

Among long-acting peptides can be cited semaglutide, dulaglutide, liraglutide, cagrilintide, albiglutide, davalintide, tirzepatide, cotadutide or MEDI-0382,, albiglutide, BI 456906 and SAR 425899.

In an embodiment, the invention relates to a composition free of protamine salt.

In another embodiment, the invention relates to a composition free of protamine salt or other positively charged peptides comprising a percentage of positively charged amino acids which is greater than or equal to 40%.

In an embodiment the composition does not contain amylin, in particular human amylin.

In an embodiment the composition does not contain salmon calcitonin (sCT).

In an embodiment the composition does not contain GLP-1, in particular human GLP-1.

In an embodiment the composition according does not contain insulin.

In an embodiment the composition does not contain grafted insulin.

In an embodiment the composition does not contain acylated insulin.

In an embodiment the composition does not contain pegylated insulin.

In an embodiment, the composition does not contain a long-acting insulin.

In an embodiment, the composition does not contain insulin glargine.

In an embodiment, the composition does not contain a prandial insulin.

In an embodiment, the composition does not contain a prandial insulin with a substitution on the A21 position.

According to an embodiment, the composition does not comprise a copolyamino acid bearing carboxylate charges and hydrophobic radicals.

According to an embodiment, the composition does not comprise a copolyamino acid consisting of more than 80 % of glutamate and aspartate residues and bearing carboxylate charges and hydrophobic radicals.

According to an embodiment, the composition does not comprise a copolyamino acid consisting of more than 80 % of glutamate and aspartate residues and bearing an acyl group comprising more than 6 carbon atoms.

In an embodiment, the composition according to the invention does not contain a copolyaminoacid consisting of glutamate or aspartate units and said copolyaminoacid bearing at least one acyl group comprising from 6 and 25 carbon atoms.

In an embodiment the composition according to the invention does not comprise an amphiphilic compound comprising a hydrophilic backbone bearing at least one acyl group comprising from 6 and 25 carbon atoms.

In an embodiment the composition according to the invention does not comprise a copolyamino acid bearing carboxylate charges and hydrophobic radicals described in patent applications WO2017211918 and WO2017211917.

In an embodiment the composition according to the invention does not comprise a copolyamino acid bearing carboxylate charges and hydrophobic radicals described in patent application WO2019/110837.

In an embodiment the composition according to the invention does not comprise a copolyamino acid bearing carboxylate charges and hydrophobic radicals described in patent application WO2019110836.

In an embodiment the composition according to the invention does not comprise a copolyamino acid bearing carboxylate charges and hydrophobic radicals described in patent application WO2019110838.

In an embodiment the composition according to the invention does not comprise a copolyamino acid bearing carboxylate charges and hydrophobic radicals described in patent application WO2020025825.

In an embodiment the composition according to the invention does not comprise a copolyamino acid bearing carboxylate charges and hydrophobic radicals described in patent application EP20217255.7.

In an embodiment the composition according to the invention does not comprise a copolyamino acid bearing carboxylate charges and hydrophobic radicals described in patent application EP20217270.6

In an embodiment, the pharmaceutical formulation comprising the composition according to the invention is not an aerosol form.

According to an embodiment the composition does not comprise long-acting glucagon suppressor.

According to an embodiment the composition does not comprise long-acting GLP-1 RA.

According to an embodiment the composition does not comprise long-acting acylated GLP-1 RA.

According to an embodiment the composition does not comprise long-acting GLP-1 RA acylated with an acid comprising an alkyl chain of at least 10 carbon atoms.

In an embodiment, the composition does not comprise amylin.

According to an embodiment the composition does not comprise long-acting amylin RA.

According to an embodiment the composition does not comprise long-acting acylated amylin RA.

According to an embodiment the composition does not comprise long-acting amylin RA acylated with an acid comprising an alkyl chain of at least 10 carbon atoms.

According to an embodiment the composition does not comprise long-acting glucagon RA.

According to an embodiment the composition does not comprise long-acting acylated glucagon RA.

According to an embodiment the composition does not comprise long-acting glucagon RA acylated with an acid comprising an alkyl chain of at least 10 carbon atoms.

In an embodiment, the invention relates to a pharmaceutical composition comprising compositions as defined above.

The invention also concerns a container comprising a pharmaceutical formulation according to the invention.

In one embodiment the container is a cartridge.

In one embodiment the container is a vial.

The invention also concerns a method of preparation of stable injectable compositions.

In one embodiment, the composition according to the invention also comprise buffers.

In one embodiment, the composition according to the invention comprises buffers at concentrations from 0 to 100 mM.

In one embodiment, the composition according to the invention comprises buffers at concentrations from 15 to 50 mM.

In one embodiment, the composition according to the invention comprise a buffer chosen from the group consisting of a phosphate buffer, acetate buffer, Tris (trishydroxymethyl)aminomethane and sodium citrate.

In one embodiment, the buffer is sodium phosphate.

In one embodiment, the buffer is Tris (trishydroxymethyl)aminomethane.

In one embodiment, the buffer is sodium citrate.

In one embodiment, the buffer is sodium acetate.

In one embodiment, the composition according to the invention comprises at least one antioxydant.

In one embodiment, the antioxydant is methionine.

In one embodiment, the concentration in antioxydant is comprised from 0.5 to 20 mM.

In one embodiment, the concentration in antioxydant is comprised from 2 to 10 mM.

In one embodiment, the composition according to the invention also comprise at least one preservative.

In one embodiment, the preservative is chosen from the group consisting of m-cresol and phenol, alone or in mixture.

In one embodiment, the concentration of the preservative is comprised from 10 to 50 mM.

In one embodiment, the concentration of the preservative is comprised from 10 to 40 mM.

In one embodiment, the concentration of the preservative is comprised from 20 to 30 mM.

In one embodiment, the composition according to the invention further comprise at least a surfactant.

In one embodiment, the surfactant is chosen in the group consisting of propylene glycol and polysorbates such as polysorbate 20 also called Tween^{®}20.

In one embodiment, the surfactant is chosen in the group of polysorbates.

In one embodiment, the concentration in polysorbate is comprised from 4 to 20 µM.

Composition according to the invention may further comprise additives such as tonicity agents.

In one embodiment, the tonicity agents are chosen in the group consisting of glycerine, sodium chloride, mannitol and glycine.

Composition according to the invention may further comprise all excipients compliant with pharmacopoeias and compatible with the insulins used at the customary concentrations.

The invention also relates to a pharmaceutical formulation according to the invention, obtained by drying and/or lyophilization.

The composition may be used for preventing or treating overweight, obesity and related diseases.

By "preventing or treating overweight, obesity and related diseases" is meant preventing or treating obesity, NASH, NAFLD, overweight, reducing body weight and/or food intake, or inducing energy expenditure or satiety.

In an embodiment the composition is used for preventing or treating obesity.

In an embodiment the composition is used for preventing or treating overweight.

In an embodiment the composition is used for preventing or treating NASH.

In an embodiment the composition is used for preventing or treating NAFLD.

In an embodiment the composition is used for reducing body weight.

In an embodiment the composition is used for reducing food intake.

In an embodiment the composition is used for inducing satiety.

In an embodiment the composition is used for inducing energy expenditure.

In another embodiment, the composition is used for preventing or treating neurodegenerative diseases.

In another embodiment, the composition is used for preventing or treating Type 2 Diabetes.

By "treating overweight, obesity and related diseases" is meant treating obesity, NASH, NAFLD, overweight, reducing body weight and/or food intake, or inducing energy expenditure or satiety.

The composition may be used for treating obesity and related diseases.

In an embodiment the composition is used for treating obesity.

In an embodiment the composition is used for treating overweight.

In an embodiment the composition is used for treating NASH.

In an embodiment the composition is used for treating NAFLD.

In another embodiment, the composition is used for treating Type 2 Diabetes.

In another embodiment, the composition is used for treating neurodegenerative disease. In particular the neurodegenerative disease is parkinson disease or alzheimer diseases.

According to an embodiment, the invention relates to a scheme of administration of a composition as defined above.

According to a further embodiment, the composition is delivered by a device as defined in the instant specification.

In an embodiment the scheme of administration comprises periods where the delivery is decreased or stopped.

In a further embodiment the scheme of administration comprises modulation of the delivered dose.

In a further embodiment the scheme of administration comprises modulation of the delivered dose, such as increasing and decreasing the delivery, or even stop and restart periods.

In a further embodiment the scheme of administration comprises modulation periods that allow a modulation of the daily dose.

In a further embodiment the scheme of administration comprises modulation periods that allow a decrease of the daily dose until it is discontinued, temporarily or definitively.

In a further embodiment the scheme of administration comprises stop and restart periods.

In a further embodiment the scheme of administration comprises modulation of the delivered dose during the day without changing the total daily dose.In an embodiment the patient decides for the decrease of the delivery.

In another embodiment the decrease or total stop of delivery is done automatically for certain periods, for exemple during the night or part of the night.

The increase or the decrease of the delivery may be instantaneous or can reach the desired value through steps or through a ramp.

In an embodiment the patient may stop or decrease the delivery of the composition due to undesired side effects due to the composition or to take a short break of the treatment, for example to have a "normal" eating behaviour. These periods may help the patient to improve his state of mind and then to improve his compliance on the long-term of the treatment.

The administration of the composition may be modulated.

The modulation may be a decrease of the administration, for example by 10, 20, 30, 40, 50, 60, 70, 80, 90 or even 100 %.

The modulation may be an increase of the administration, for example by 10, 20, 30, 40, 50, 60, 70, 80, 90, 100, 150, 200, 300, 400, 500, 600, 700, 800, 900, or 1000 %.

By increase or decrease of the composition administered it may be the administration per hour or per day.

The modulation may last at least 1, 2, 3, 6, 12, 24, 36 or 48h.

The modulation may last at most 2, 4, 8, 12, 24, 36, 48 or 72h.

The period between a stop and a restart can the same than those defined for modulation above.

For example the administration may stop for a few hours over night or during the sleeping time of the patient and restarts in the early morning or a short time before the patient wakes up.

The administration may be diminished or stopped at 10pm, 11pm or 12pm and start again at 4am, 5am or 6am.

In an embodiment, the administration goes back to the rate of administration it was before the stop or the diminution.

In an embodiment, the administration starts again at a lower rate of administration than the one before the stop or diminution and goes back proggressively to the rate before the stop or diminution.

In an embodiment the modulation of the rate of delivery happens intraday.

In an embodiment this intra day modulation lasts at least 1, 2, 3, 4, 6, 8, 12, 16 h.

In an embodiment this intra day modulation lasts at most 2, 3, 4, 6, 8, 12, 16, 20 h.

In an embodiment the daily dose remains stable for a week, 2 weeks, 4 weeks, 2 months, 4 months, 6 months, a year or 2 years.

By "the daily dose remains stable" is meant the daily dose varies by less than 10%, in particular less than 5%.

In an embodiment, the daily dose may increase over 2, 4, 8 months. In particular during the beginning of the treatment until reaching the optimal daily dose.

In an embodiment, the daily dose may decrease over a period of 2, 4, 8 months. In particular before stopping the treatment.

According to an embodiment, the device delivers the composition via subcutaneous injection.

Referring to figure 2, the administering apparatus 2 can comprise:
- a housing 21
- a reservoir 22,
- a drive mechanism 23,
- a control unit 24,
- a power source 25,
- an injection set 26,
- optionally one or more sensor 27, and
- optionally an output/display 28.

The housing 21 can be of any suitable shape and size. For instance, the housing 21 may be in the shape of a square, rectangle, circle, cylinder or the like.

The housing 21 may be dimensioned so as to be comfortably associated with the patient and/or hidden from view, for instance, within the clothes of the patient. For instance, the housing 21 may have width and height of about 1 to about 15 centimeters, and a thickness of about 0.5 to about 2 centimeters. As used herein, the term "about" in the context of a given value or range refers to a value or range that is within 20%, preferably within 10%, and more preferably within 5% of the given value or range.

The materials of the housing 21 may vary as well. For instance, the housing 21 may be made of metal or plastic.

The housing 21 is designed to be worn by the patient. In one embodiment, the housing 21 includes gripping means (not represented) for fastening the administering apparatus on a patient's cloth, such as a belt. In another embodiment, the housing 21 includes an adhesive patch for adhesively attaching the administering apparatus to an infusion site on the patient's skin.

The reservoir 22 is adapted to store the composition 1 to be administered to the patient.

The reservoir 22 may be a single-use reservoir or a refillable reservoir.

The reservoir 22 have any suitable shape and size configured for receiving, storing, and releasing the composition 1. The reservoir 22 may be a container formed by a flexible material or a rigid material. The reservoir 22 may have a capacity of about 1 to about 10 milliliters.

In some embodiments, the reservoir 22 is integrated to the housing 21. In other embodiments, the reservoir 22 is separated from the housing 21. In that case, the housing 21 includes a receiving slot for reversibly attaching/detaching the reservoir 22 to the housing 21.

The drive mechanism 23 is configured for pumping the composition 1 contained in the reservoir 22 to an outlet port of the housing 21 connected to the injection set 26.

The drive mechanism 23 can be a pump, such as a peristaltic drive pump, or any other suitable type of drive mechanism known by the skilled person for pumping a fluid from a container to an outlet port.

The control unit 24 allows driving the drive mechanism 23, exchanging data with the supervisory terminal 3.

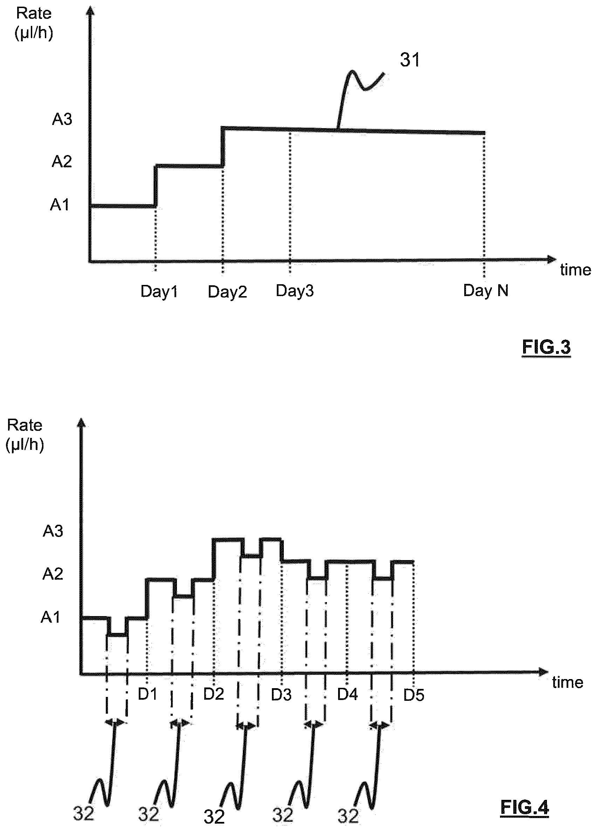

Referring to figure 3, the control unit 24 may include:
- a memory for storing one or more injection profile, one or more user parameters, one or more data measured by a sensor (or sensors) of the administering apparatus 2,
- a transceiver (wired or wireless) for transmitting/receiving information with the supervisory terminal 3,
- an alarm (visual indicator and/or audible indicator and/or tactile indicator) for providing visual/audible/tactile feedback to the patient regarding an operation of the administering apparatus 2, such as:
   ∘ the need to replace or refile the reservoir 22, or
   ∘ The need to replace or charge the power source 25, etc.
- a processor to control the overall functions of the drive mechanism 23.

The processor may also include programming that allows the processor to receive signals and/or other data from the supervisory terminal 3, or from various sensors (included as a part of the administering device 2 or used in conjunction therewith) and to store the same in the memory.

The power source 25 allows powering the administering apparatus 2 with electrical energy. The power source 25 may be composed of one or more batteries - which can be rechargeable or not. The power source 25 can be integrated within the housing 21 or separated from the housing 21 and connected to the housing 21 via electric wires.

Such power source 25 is well known in the art and will not be described in more details below.

The injection set 26 allows conveying the composition 1 into the skin of the patient.

Depending on the type of the administering apparatus 2 ("*conventional pump"* or "*patch pump"*), the injection set 26 can be integrated to or separated from the housing 21.

If the administering apparatus 2 is a "*conventional pump",* then the infusion set 26 is separated from the administering apparatus 2 and may include:
- a connector,
- a length of tubing, and
- a hub from which a cannula, in the form of a hollow metal infusion needle or flexible plastic catheter extends; advantageously, the hub can have an adhesive that retains the hub on the skin surface during use.

If the administering apparatus 2 is a *"patch pump",* then the infusion set 26 is integrated to the administering apparatus 2. Indeed, a *"patch pump"* is an integrated device that combines most or all of the fluidic components (including the fluid reservoir 22, the drive mechanism 23 and the components adapted for automatically inserting the cannula) in a single housing which is adhesively attached to an infusion site on the patient's skin, and does not require the use of a separate infusion set. Configuring the administering apparatus 2 as a *"patch pump"* allows minimizing the size of the device in order to limit the interference between the use of said device and the activities of the patient. Hence, configuring the administering apparatus 2 as a *"patch pump"* allows reducing the discomfort associated to its use for the patient.

In any case, the infusion set 26 includes a cannula for the subcutaneous delivery of the composition to the patient.

As mentioned above, the administering apparatus 2 may comprise one or more sensor 27:
- for measuring physiological parameters of the patient, such as an accelerometer, a gyroscope, a pressure sensor, a temperature sensor, a blood glucose sensor, and the like, and/or
- for monitoring the administering apparatus 2, such as a sensor measuring the amount of energy available in the power source 25, a sensor measuring the amount of composition 1 contained in the reservoir 22, a sensor detecting a malfunctioning of the drive mechanism 23 (for example due to an occlusion preventing delivery of the composition 1, etc.).

For some applications, the administering apparatus 2 can include a display screen 28.

The type of display 28 may include LCD displays, LED displays, plasma displays, OLED displays and the like. The display 28 may also be an interactive or touch sensitive screen having an input device such as a touch screen, a capacitance screen, a resistive screen or the like.

Moreover, in some embodiments, the administering apparatus 2 may additionally include data entry means - such as a keyboard or other input device known in the art - for data entry, which may be separate from the output/display 28.

The supervisory terminal 3 allows controlling the administering apparatus 2. The supervisory terminal 3 can be integrated to the administering apparatus 2, or separated from the administering apparatus 2, as shown on figure 1.

For instance, the supervisory terminal 3 can consist of one (or several) work station(s) and/or one (or several) computer(s) or may be of any other type known to one skilled in the art.

The supervisory terminal 3 can also consist of a mobile phone (such as a smartphone), an electronic tablet (such as an IPAD^{®}), a Personal Digital Assistant (or "PDA"), etc.

The supervisory terminal 3 comprises a processing unit programmed in order to:
- communicate with the administering apparatus 2,
- estimate an injection profile according to user parameters and/or physiological parameters of the patient,
- process signals measured by sensors, and/or
- display information to the patient on a display element such as a display screen.

With regard to the processing of the signals, the processing unit can be implemented in order to alert a practitioner and/or the patient if a signal measured by a sensor (or signals measured by a plurality of sensors) exceed a heath threshold representative of a risk for the health of the patient.

Advantageously, the supervisory terminal 3 can include a Graphic User Interface (GUI) allowing the patient to input user parameter(s) with regard to the administration of the composition 1.

Indeed, the device can be configured to adapt the injection profile of the composition 1 according to one (or a plurality of) user parameter(s).

The supervisory terminal 3 may comprise a database including a plurality of different injection profiles 31, each infusion profile 31 defining one rate, or multiple rates over time for the continuous administration of the composition 1 to the patient. An example of infusion profile is given on figure 3. This injection profile is composed of different stages of rates increasing as a function of time. The skilled person will appreciate that the injection profile can also consist in a rate evolution according to a substantially constant rate ramp up, with the gradient of this ramp being on the order of 0.1 to 100 % per hour. This allows a progressive increase of the amount of composition that is injected to the patient in order to adjust his body, thus limiting the side effects, such as nausea or diarrhea, felt by the patient.

Advantageously, the administering of the composition can be implemented by injecting a plurality of successive bolus doses several times (per hour or per day). In an embodiment, the volume of each bolus dose being in a range of 0.01 to 10 µL, more preferably in a range of 0.05 to 5 µL, even more preferably in a range of 0.05 to 2 µL, especially preferably in a range of 0.05 to 1 µL.

As discussed above, the injection of the composition 1 can induce several side effects to the patient, such as nausea. These side effects are more pronounced at the beginning of the treatment (i.e., the three or four first weeks of administration of the composition). Moreover, these side effects can vary from one patient to another. This is why the device can be configured to take into account a feeling of the patient in order to determine an injection profile 31 suitable with the comforting sensations induced by the composition 1.

In particular, the supervisory terminal 3 may be configured to extract one injection profile of the plurality of injection profiles contained in the database according to one (or more than one) parameter(s) as will be discussed in more details below. Alternatively (or in combination), the supervisory terminal 3 can be configured to modify a current injection profile defined for a patient according to one (or more than one) parameter(s), for instance by increasing or decreasing a rate of the injection profile at specific moments of the day.

Moreover, in some cases, the satiety sensation does not need to be provided to the patient every time. For instance, when a patient is sleeping the injection rate of the composition 1 can be decreased in order to limit the amount of composition 1 administered continuously to the patient. This allows optimizing the consumption of the composition 1 contained in the reservoir 22 in order to:
- limit the risks associated with the administration of the composition 1 to the patient,
- extend the operating life of the device .

Different embodiments allowing an adjustment of the injection profile 31 according to a single parameter are discussed below. The skilled person will appreciate that the different parameters can be combined in order to adjust an injection profile 31.

In one embodiment, the device allows adapting the injection profile 31 according to a level of comfort defined by the patient.

More particularly, when the patient is supplied with the device , the supervisor terminal 3 extracts a first predetermined injection profile from the database and transmits said first injection profile to the control unit 24 of the administering apparatus 2.

The control unit 24 drives the drive mechanism 23 according to the first injection profile for continuously injecting the correct amount of composition 1 to the patient.

If the patient wishes to inform the supervisory terminal 3 of his feeling in response to the injection of the composition 1, the patient can input a level of comfort on the GUI of the supervisory terminal 3. Alternately, the supervisory terminal 3 can send a message to the patient requesting him to notify the supervisory terminal 3 of his level of comfort on the GUI of the supervisory terminal 3.

The level of comfort can be defined by a plurality of values representative of a sensation of nausea felt by the patient. For instance, the sense of nausea can be defined on a "*1 to 5*" scale:
- a value of "*1*" corresponding to a state where the patient does not feel side effect(s), such as nausea or diarrhea, and
- a value of "*5*" corresponding to a state where the patient does not feel fine, related to side effects.

Alternately or in combination, the level of comfort can be defined by a plurality of values representative of a sensation of hunger felt by the patient. For instance, the sense of hunger can be defined on a *"1 to 5*" scale:
- a value of "*1*" corresponding to a state where the patient does not feel hungry, and
- a value of "*5*" corresponding to a state where the patient feels hungry.

The skilled person will appreciate that other information can be used for notifying the supervisory terminal 3 with the level of comfort of the patient, such as a sense of stress, a sense of irritation, etc.

In response to the level of comfort defined by the user, the device can adapt the injection rate of the composition 1. In particular, the supervisory terminal 3 can estimate whether the injection profile determined for the patient needs to be amended or replaced.

For instance, if the patient informs the supervisory terminal 3 that he feels side effects, the supervisory terminal 3 can:
- determine a second injection profile wherein the injection rate(s) of the composition is (are) lower, and
- transmit this second injection profile to the control unit 24 in order to decrease the amount of composition 1 injected subcutaneously to the patient.

Since the composition 1 is a short-acting composition, the patient will feel better rapidly.

Alternatively, the supervisory terminal 3 can modify the rate of the current injection profile set to the patient by decreasing the rates defined in the injection profile for a given period of time 32. In some embodiments, this given period 32 of rate decrease can be automatically applied for each day of the injection profile (as shown on figure 4) so that the patient does not have to inform the supervisory terminal 3 of its feeling each day of the treatment.

On the contrary, if the patient informs the supervisory terminal 3 that he feels hungry, the supervisory terminal 3 can:
- extract a third injection profile from the database (or compute a third injection profile) where the injection rate(s) is (are) higher, and
- transmit this third injection profile to the control unit 24 in order to increase the amount of composition 1 administered to the patient.

If the supervisory terminal 3 compute the third injection profile, a comparison of the computed rate(s) with a predetermined maximum threshold (representative of a risk for the patient's health) can be implemented. Again, any increase of an injection rate for a given period of time of a day can be applied to the other days of the injection profile.

Advantageously in some embodiments, levels of comfort defined by the patient at different times of the day can be used by the supervisory terminal 3 in order to adapt the injection profile. In particular, the amount of composition 1 administered to the patient can be decreased at one (or plural) given time(s) of a current day, if the patient informed the supervisory terminal 3 of a side effect feeling at said given time(s) of a previous day. This allows dynamically adapting the treatment to the patient.

Conversely, the amount of composition administered to the patient can be increased at one (or plural) given time(s) of a current day, if the patient did not inform the supervisory terminal 3 of a nausea feeling at said given time(s) of a previous day. This allows increasing the effect of the treatment, for example improving the satiety feeling, at specific time of the day (for instance just before breakfast/lunch/diner time, or at specific period(s) defined by the user as being more critical with regard to his eating pattern, etc.) in order to improve the efficency of the solution according to the invention.

In another embodiment, the device allows adjusting the injection profile according to the patient's activity.

More particularly, when the patient is supplied with the device , the supervisor terminal 3 can modify the injection profile according to the motions of the patient.

For instance, if the administering apparatus 2 includes one (or more) sensor(s) ― such as a gyroscope of an accelerometer ― the control unit 24 can detect when the patient is in an active state or in a passive state, and can communicate this information to the supervisor terminal 3:
- when the patient is detected as being in an active state, the supervisor terminal 3 determines a first injection profile defining at least one first rate, and transmits this first injection profile to the control unit 24 for driving the drive mechanism 23,
- when the patient is detected as being in a passive state (for instance the patient is sleeping), the supervisor terminal 3 determines a second injection profile defining at least one second rate smaller than the first rate, and transmits this second injection profile to the control unit 24 for driving the drive mechanism 23.

In further other embodiments, the device may be configured to adjust the injection profile according to other parameters, such as the weight of the patient, or the occurrence of a special event in the life of the patient (a wedding, a birthday, etc.) during which the patient wants to feel "normal", or on the contrary wants to increase the effect of its treatment.

For instance, the patient can input his weight and/or the occurrence of a special event on the GUI of the supervisory terminal 3. In response to these user parameters, the supervisory terminal 3 can adjust the injection profile in order to decrease (or increase) the amount of composition 1 to be continuously administered to the patient subcutaneously.

As mentioned above, the administering apparatus 2 of the device can include a lot of features, in particular when the supervisory terminal 3 is incorporated within the administering apparatus 2 (memory, transmitter/receiver, alarm, input device, processor, output/display, graphic user interface, drive mechanism, processing unit, sensors, etc.).

However, for some applications, the administering apparatus 2 can be more basic. This allows reducing the size and the complexity of use of the administering apparatus 2.

In one embodiment the device can be composed of a set of different administering apparatuses 2, each administering apparatus 2 of the set being preprogrammed with a single predetermined injection profile that is different from the other administering apparatus 2 of the set.

In this case, each administering apparatus 2 comprises a housing 21, a reservoir 22, a drive mechanism 23, a basic control unit 24, a power source 25, and an injection set 26. The basic control unit 24 of each administering apparatus 2 comprises: a memory storing a single predetermined injection profile, optionally an alarm, optionally data entry means, and a processor controlling the drive mechanism 23 according to the preprogrammed injection profile stored in the memory of the administering apparatus 2.

With such a basic administering apparatus 2, the patient can act by stopping (ON/OFF) the injection of the composition 1 for a given period of time (for example 1 hour, 2 hours, 1 day, etc.), for instance when he feels side effects. To this end, the administering apparatus 2 can include an actuation element ― such as a button ― that allows deactivating the administering apparatus 2 for a short period of time when said actuation element is pressed. After this short time period, the administering apparatus 2 is automatically reactivated and the continuous administering of the composition 1 restarts. In some embodiments of the invention, the given period of time can be defined by the patient (for example by using data entry means of the administering apparatus 2). Alternately to the stopping of the injection procedure, the patient can request a decrease of the injection rate of the composition (for instance a decrease of 10%, 20%, etc.) by using the data entry means. The skilled person will appreciate that the decreasing (or stopping) of the injection procedure can be implemented progressively, according to different stages of rates decreasing as a function of time, or according to a substantially constant rate ramp down.

Advantageously, if the patient stops the injection of the composition 1 for a given period of time that exceeds a time threshold, the administering apparatus 2 can be configured to:
- activate an alarm (visuable and/or tactile and/or audible stimuli) in order to warn the patient that the treatment has been interrupted for a too long period of time, and that the injection of the composition must be restarted; and/or to
- automatically restart the injection of the composition.

The restart of the injection of the composition can be implemented according to different stages of rates increasing as a function of time, or according to a substantially constant rate ramp up in order to progressively increase the amount of composition that is injected to the patient.

Before each replacement of the administering apparatus 2 (for instance when the reservoir is empty), a practitioner determines whether an identical administering apparatus 2, or an administering apparatus 2 designed with another injection profile (defining a higher/lower injection rate) must be provided to the patient.

The above disclosed device allows an efficient treatment for a patient while limiting the side effects associated with the administration of the composition 1.

Advantageously, the different injection profiles (preprogrammed or calculated by the supervisory terminal 3) are defined in a range of rates that are:
- above an efficiency threshold, and preferably
- below a discomfort threshold,
in order to provide the patient with a solution that does not cause a problem of tolerability of the medication.

The skilled person will have understood that many modifications may be provided to the invention described above without materially departing from the new teachings and advantages described here.

### EXAMPLES

### Part A - Synthesis of the hydrophobic copolyamino acids

The intermediate hydrophobic compounds Hy and the copolyamino acids disclosed below can be synthesized according to the procedures described in WO2019110773, WO2019110837, WO2019110788, WO2019110797, WO2019110836, WO2019110774, WO202011533 and WO2020245470.
i) Co-polyamino acids according to formula XXXa

**Table 1: Copolyamino acids according to formula XXXa ii) Co-polyamino acids according to formula XXXb**

| No. | CO-POLYAMINO ACIDS BEARING CARBOXYLATE CHARGES AND HYDROPHOBIC RADICALS |
|---|---|
| B1 | 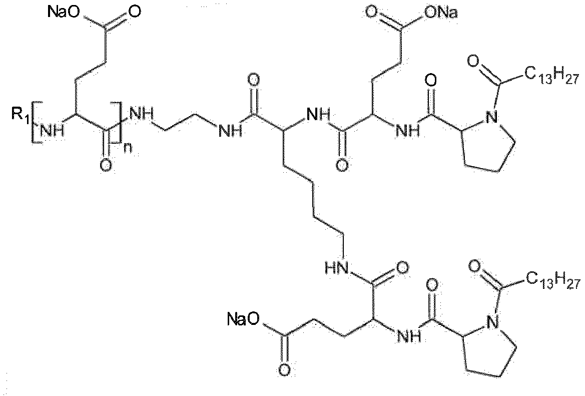 |
| | i=0,043, DP = 23 |
| | R₁ = H or pyroglutamate |
| B2 | 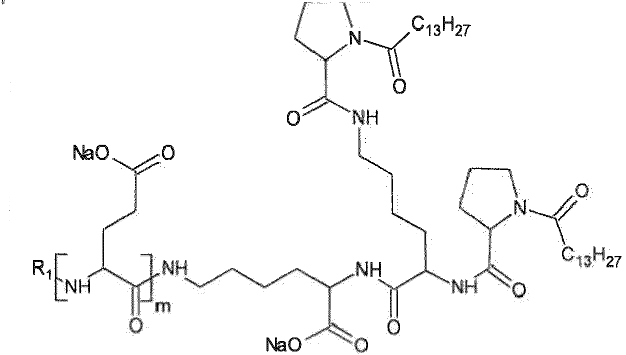 |
| | i = 0.038, DP = 26 |
| | R₁ = H or pyroglutamate |
| B3 | 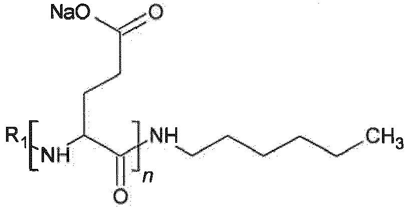 |
| | i = 0,042, DP = 24 |
| | R₁ = |
| | 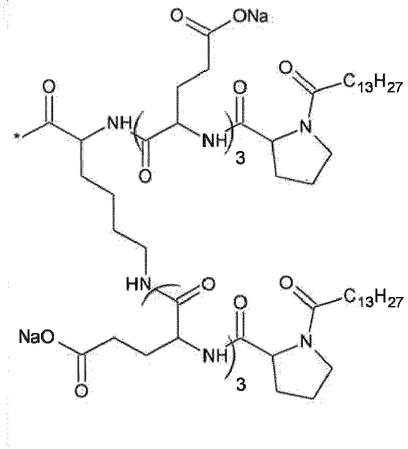 |
| B4 | 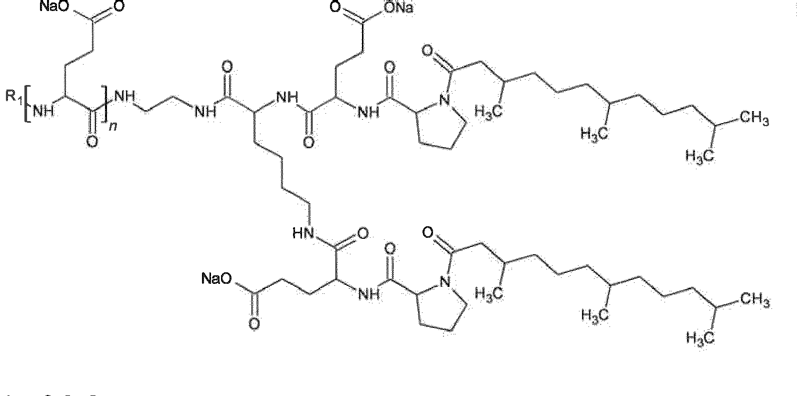 |
| | i = 0.042, DP = 24 |
| | R₁ = H or pyroglutamate |
| B5 | 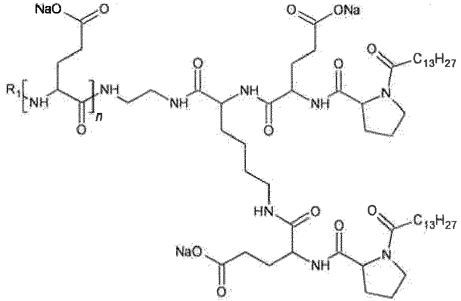 |
| | i = 0.1, DP = 10 |
| | R₁ = H or pyroglutamate |
| B6 | 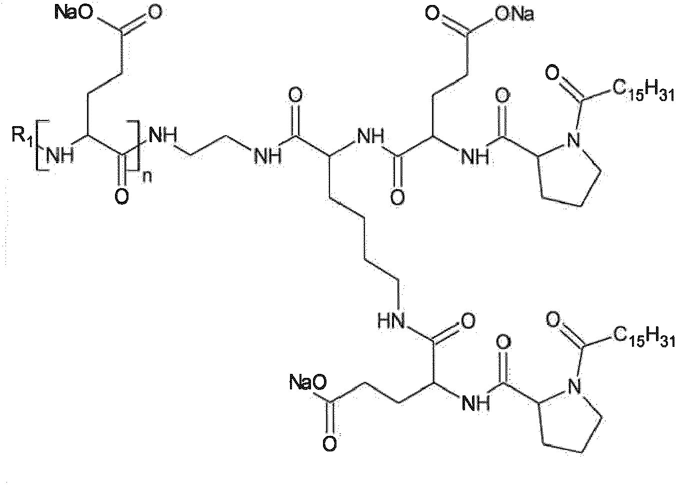 |
| | i = 0,042, DP = 24 |
| | R₁ = H or pyroglutamate |
| B7 | 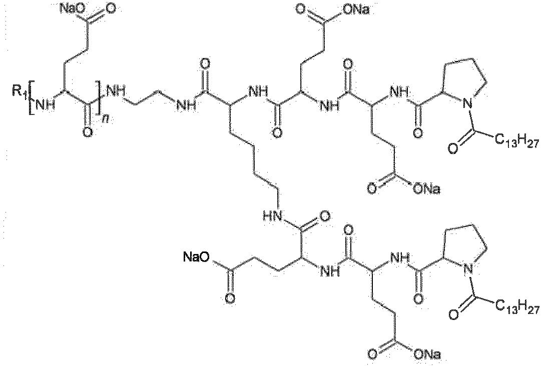 |
| | i=0,043, DP = 23 |
| | R₁ = H or pyroglutamate |

**Table 2: Copolyamino acids according to formula XXXb**

| | |
|---|---|
| B8 | 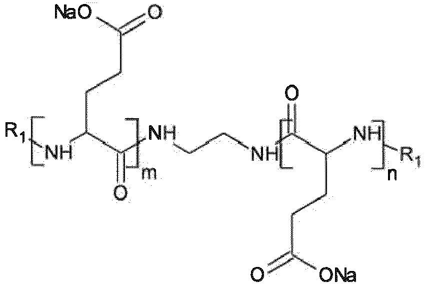 |
| | i= 0,0833, DP (m + n) = 24 |
| | R₁ = H, pyroglutamate or |
| | 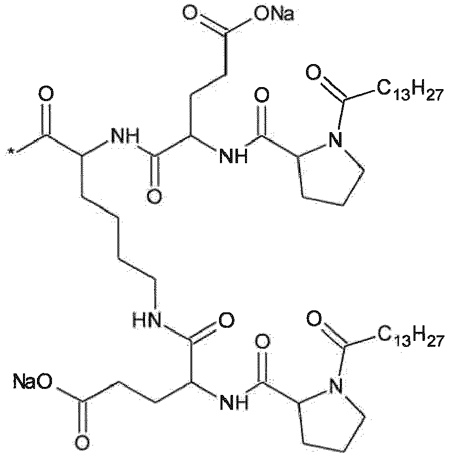 |
| B9 | 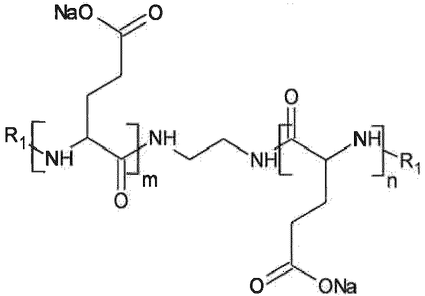 |
| | i= 0,079, DP (m + n) = 24 |
| | R₁ = H, pyroglutamate or |
| | 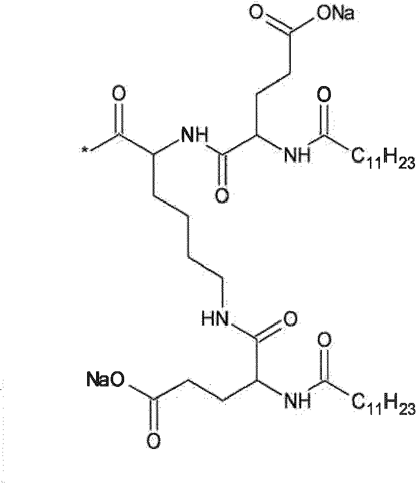 |
| B10 | 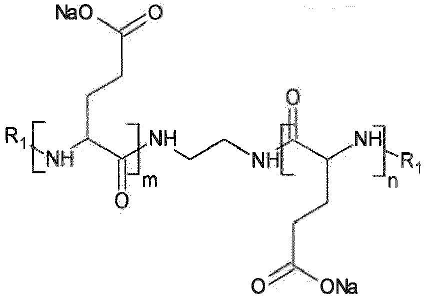 |
| | i= 0,072, DP (m + n) = 24 |
| | R₁ = H, pyroglutamate or |
| | 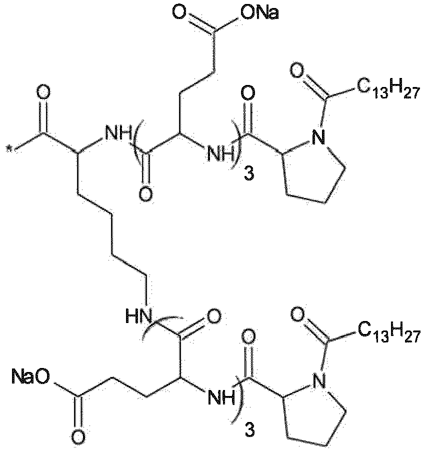 |
| B11 | 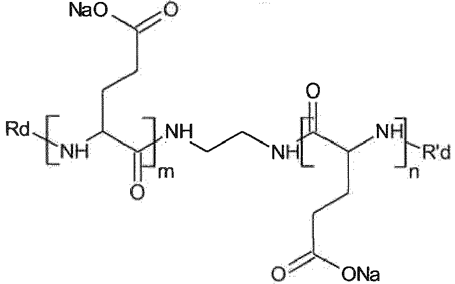 |
| | i=0,083, DP (m + n) = 24 |
| | R_{d}, R_{'d} = H, pyroglutamate or |
| | 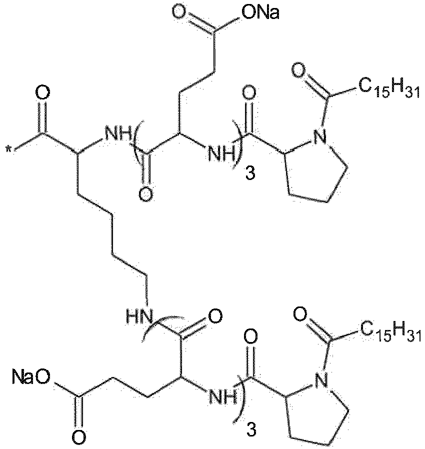 |

### Part B ― Compositions

### 1 - Compositions comprising pramlintide and a GLP-1 receptor agonist (GLP-1 RA)

In the following examples, pramlintide, exenatide, lixisenatide, human glucagon and dasiglucagon have been obtained through peptide synthesis.

Example C7 : Preparation of solutions of pramlintide in combination with exenatide or lixisenatide at pH 4.

A stock solution of pramlintide at pH 2.0 - 3.0 was prepared using pramlintide as a powder, water was added and hydrochloric acid was used for pH adjustment.

A stock solution of exenatide or lixisenatide was prepared using exenatide or lixisenatide as powder, water was added and the pH was not adjusted.

In a container, stock solutions of excipients were added to water: m-cresol, mannitol or glycerol, and acetate buffer. The concentrations of m-cresol, mannitol, glycerol and acetate buffer were chosen to reach the desired concentrations in the compositions described in table 3. Then were added in the following order: exenatide or lixisenatide stock solution then pramlintide stock solution. The pH was adjusted to 3.7 - 4.0 by addition of a solution of hydrochloric acid or of sodium hydroxide.

The compositions obtained are detailed in table 3. The pH was adjusted to 4 by addition of a solution of hydrochloric acid or of sodium hydroxide.

**Table 3: compositions according to the invention C7-1 to C7-25 and C8-1 to C8-24.**

| Compo sition | Pramlin tide (mg/mL) | Exenatide (µg/mL) | Lixisenatide (µg/mL) | Excipients |
|---|---|---|---|---|
| C7-1 | 0.6 | 24 | - | m-cresol (20 mM), Acetate buffer (20 mM), Mannitol (250 mM) |
| C7-2 | 0.6 | 36 | - | m-cresol (20 mM), Acetate buffer (20 mM), Mannitol (250 mM) |
| C7-3 | 0.6 | 48 | - | m-cresol (20 mM), Acetate buffer (20 mM), Mannitol (250 mM) |
| C7-4 | 0.6 | 60 | - | m-cresol (20 mM), Acetate buffer (20 mM), Mannitol (250 mM) |
| C7-5 | 0.6 | 72 | - | m-cresol (20 mM), Acetate buffer (20 mM), Mannitol (250 mM) |
| C7-6 | 1 | 40 | - | m-cresol (20 mM), Acetate buffer (20 mM), Mannitol (250 mM) |
| C7-7 | 1 | 60 | - | m-cresol (20 mM), Acetate buffer (20 mM), Mannitol (250 mM) |
| C7-8 | 1 | 80 | - | m-cresol (20 mM), Acetate buffer (20 mM), Mannitol (250 mM) |
| C7-9 | 1 | 100 | - | m-cresol (20 mM), Acetate buffer (20 mM), Mannitol (250 mM) |
| C7-10 | 1 | 120 | - | m-cresol (20 mM), Acetate buffer (20 mM), Mannitol (250 mM) |
| C7-11 | 2 | 80 | - | m-cresol (20 mM), Acetate buffer (20 mM), Mannitol (250 mM) |
| C7-12 | 2 | 120 | - | m-cresol (20 mM), Acetate buffer (20 mM), Mannitol (250 mM) |
| C7-13 | 2 | 160 | - | m-cresol (20 mM), Acetate buffer (20 mM), Mannitol (250 mM) |
| C7-14 | 2 | 200 | - | m-cresol (20 mM), Acetate buffer (20 mM), Mannitol (250 mM) |
| C7-15 | 2 | 240 | - | m-cresol (20 mM), Acetate buffer (20 mM), Mannitol (250 mM) |
| C7-16 | 3 | 120 | - | m-cresol (20 mM), Acetate buffer (20 mM), Mannitol (250 mM) |
| C7-17 | 3 | 180 | - | m-cresol (20 mM), Acetate buffer (20 mM), Mannitol (250 mM) |
| C7-18 | 3 | 240 | - | m-cresol (20 mM), Acetate buffer (20 mM), Mannitol (250 mM) |
| C7-19 | 3 | 300 | - | m-cresol (20 mM), Acetate buffer (20 mM), Mannitol (250 mM) |
| C7-20 | 3 | 360 | - | m-cresol (20 mM), Acetate buffer (20 mM), Mannitol (250 mM) |
| C7-21 | 4 | 160 | - | m-cresol (20 mM), Acetate buffer (20 mM), Mannitol (250 mM) |
| C7-22 | 4 | 240 | - | m-cresol (20 mM), Acetate buffer (20 mM), Mannitol (250 mM) |
| C7-23 | 4 | 320 | - | m-cresol (20 mM), Acetate buffer (20 mM), Mannitol (250 mM) |
| C7-24 | 4 | 400 | - | m-cresol (20 mM), Acetate buffer (20 mM), Mannitol (250 mM) |
| C7-25 | 4 | 460 | - | m-cresol (20 mM), Acetate buffer (20 mM), Mannitol (250 mM) |
| C8-1a | 0.6 | - | 60 | m-cresol (20 mM), Acetate buffer (20 mM), Mannitol (250 mM) |
| C8-1b | 0.6 | - | 90 | m-cresol (20 mM), Acetate buffer (20 mM), Mannitol (250 mM) |
| C8-2 | 0.6 | - | 120 | m-cresol (20 mM), Acetate buffer (20 mM), Mannitol (250 mM) |
| C8-3 | 0.6 | - | 150 | m-cresol (20 mM), Acetate buffer (20 mM), Mannitol (250 mM) |
| C8-4 | 0.6 | - | 180 | m-cresol (20 mM), Acetate buffer (20 mM), Mannitol (250 mM) |
| C8-5 | 1 | - | 100 | m-cresol (20 mM), Acetate buffer (20 mM), Mannitol (250 mM) |
| C8-6 | 1 | - | 150 | m-cresol (20 mM), Acetate buffer (20 mM), Mannitol (250 mM) |
| C8-7 | 1 | - | 200 | m-cresol (20 mM), Acetate buffer (20 mM), Mannitol (250 mM) |
| C8-8 | 1 | - | 250 | m-cresol (20 mM), Acetate buffer (20 mM), Mannitol (250 mM) |
| C8-9 | 1 | - | 300 | m-cresol (20 mM), Acetate buffer (20 mM), Mannitol (250 mM) |
| C8-10 | 2 | - | 200 | m-cresol (20 mM), Acetate buffer (20 mM), Mannitol (250 mM) |
| C8-11 | 2 | - | 300 | m-cresol (20 mM), Acetate buffer (20 mM), Mannitol (250 mM) |
| C8-12 | 2 | - | 400 | m-cresol (20 mM), Acetate buffer (20 mM), Mannitol (250 mM) |
| C8-13 | 2 | - | 500 | m-cresol (20 mM), Acetate buffer (20 mM), Mannitol (250 mM) |
| C8-14 | 2 | - | 600 | m-cresol (20 mM), Acetate buffer (20 mM), Mannitol (250 mM) |
| C8-15 | 3 | - | 300 | m-cresol (20 mM), Acetate buffer (20 mM), Mannitol (250 mM) |
| C8-16 | 3 | - | 450 | m-cresol (20 mM), Acetate buffer (20 mM), Mannitol (250 mM) |
| C8-17 | 3 | - | 600 | m-cresol (20 mM), Acetate buffer (20 mM), Mannitol (250 mM) |
| C8-18 | 3 | - | 750 | m-cresol (20 mM), Acetate buffer (20 mM), Mannitol (250 mM) |
| C8-19 | 3 | - | 900 | m-cresol (20 mM), Acetate buffer (20 mM), Mannitol (250 mM) |
| C8-20 | 4 | - | 400 | m-cresol (20 mM), Acetate buffer (20 mM), Mannitol (250 mM) |
| C8-21 | 4 | - | 600 | m-cresol (20 mM), Acetate buffer (20 mM), Mannitol (250 mM) |
| C8-22 | 4 | - | 800 | m-cresol (20 mM), Acetate buffer (20 mM), Mannitol (250 mM) |
| C8-23 | 4 | - | 1000 | m-cresol (20 mM), Acetate buffer (20 mM), Mannitol (250 mM) |
| C8-24 | 4 | - | 1200 | m-cresol (20 mM), Acetate buffer (20 mM), Mannitol (250 mM) |

### 2 - Compositions comprising pramlintide, a GLP-1 receptor agonist (GLP-1 RA), and a glucagon receptor agonist (glucagon RA).

Example C9: Preparation of compositions comprising pramlintide, a GLP-1 RA and/or a glucagon RA.

A stock solution of pramlintide at pH 2.0 ― 3.0 was prepared using pramlintide as a powder, water was added and hydrochloric acid was used for pH adjustment.

A stock solution of GLP-1 RA was prepared using a GLP-1 RA as powder, water was added and the pH was not adjusted.

A stock solution of glucagon receptor agonist at pH 2.5 - 3.5 was prepared using glucagon RA as powder, water was added and hydrochloric acid was used for pH adjustment.

In a container, stock solutions of excipients were added to water: m-cresol, mannitol or glycerol, and acetate buffer. The concentrations of m-cresol, mannitol, glycerol and acetate buffer were chosen to reach the desired concentrations in the compositions described in table 4. Then were added in the following order: GLP-1 RA stock solution, glucagon RA stock solution and pramlintide stock solution. The pH was adjusted to 3.7 - 4.0 by addition of a solution of hydrochloric acid or of sodium hydroxide.

The compositions obtained are detailed in table 4. The pH was adjusted to 4 by addition of a solution of hydrochloric acid or of sodium hydroxide.

**Table 4: Compositions comprising pramlintide, a GLP-1 RA and/or a glucagon RA prepared according to example C9**

| Compo sition | Pramlinti de (mg/mL) | GLP-1 RA (µg/mL) | Glucagon RA (mg/mL) | Molar ratio glucagon RA / GLP-1 RA | Excipients |
|---|---|---|---|---|---|
| C9-1 | 1.5 | Exenatide (200) | 0 | - | m-cresol (25 mM), Acetate buffer (30 mM), Mannitol (236 mM) |
| C9-2 | 1.5 | Lixisenati de (670) | 0 | - | m-cresol (25 mM), Acetate buffer (30 mM), Glycerol (184 mM) |
| C9-3 | 0 | Exenatide (100) | Dasigluca gon (1) | 12.4 | m-cresol (25 mM), Acetate buffer (30 mM), Glycerol (184 mM) |
| C9-4 | 0 | Lixisenati de (330) | Dasigluca gon (1) | 4.4 | m-cresol (25 mM), Acetate buffer (30 mM), Glycerol (184 mM) |
| C9-5 | 1.5 | 0 | Dasigluca gon (1) | - | m-cresol (25 mM), Acetate buffer (30 mM), Glycerol (184 mM) |
| C9-6 | 1.5 | Exenatide (200) | Dasigluca gon (1) | 6.2 | m-cresol (25 mM), Acetate buffer (30 mM), Glycerol (184 mM) |
| C9-7 | 1.5 | Lixisenati de (330) | Dasigluca gon (1) | 4.4 | m-cresol (25 mM), Acetate buffer (30 mM), Glycerol (184 mM) |

Compositions according to the invention allowed the formation of a co-formulation of pramlintide and/or GLP-1 RA and/or dasiglucagon at acidic pH.

### Example C10: Compositions comprising a GLP-1 RA and a glucagon RA at neutral pH.

A solution of exenatide was prepared using exenatide as powder. Water was added and the pH was not adjusted. This solution was gently stirred until the solutions was visually free of particulate matter.

A solution of dasiglucagon at pH 2.5 - 3.5 was prepared using dasiglucagon as powder, water was added and hydrochloric acid for pH adjustment. After complete dissolution of dasiglucagon, this solution was then adjusted to pH 7 by addition of sodium hydroxide and gently stirred until the solution was visually free of particulate matter.

In a container, stock solutions of excipients are added to water: m-cresol and glycerol. The concentrations of m-cresol and glycerol were chosen to reach the desired concentrations in the compositions described in table 5. Then were added in the following order: the solution of dasiglucagon and a solution of exenatide. If necessary, the pH was adjusted to 7 by addition of a solution of hydrochloric acid or sodium hydroxide.

**Table 5: Compositions comprising a GLP-1 RA and a glucagon RA prepared according to example C10.**

| Composition | exenatide (µg/mL) | dasiglucagon (mg/mL) | Molar ratio glucagon RA / GLP-1 RA | Excipients | pH |
|---|---|---|---|---|---|
| C10-1 | 200 | 1 | 6.2 | m-cresol (29 mM) Glycerol (174 mM) | 7 |

Compositions according to the invention allowed the formation of a co-formulation of exenatide and dasiglucagon at neutral pH.

### Example C11: Solutions comprising pramlintide, a GLP-1 RA and a glucagon RA in DMSO.

A solution of pramlintide at pH 2.0 - 3.0 was prepared using pramlintide as a powder, water was added and hydrochloric acid was used for pH adjustment.

Solutions of exenatide or lixisenatide were prepared using exenatide or lixisenatide as powder, water was added and the pH was not adjusted.

A solution of glucagon or dasiglucagon at pH 2.5 - 3.5 was prepared using glucagon or dasiglucagon as powder, water was added and hydrochloric acid was used for pH adjustment.

In a container, DMSO was added to water. The quantity of DMSO was chosen to reach the desired proportion in the compositions described in table 6. Then were added in the following order: the solution of exenatide or lixisenatide, a solution of glucagon or dasiglucagon and a solution of pramlintide. The total quantity of hydrochloric acid was chosen to reach the desired concentration in the compositions described in table 6.

**Table 6: Compositions comprising pramlintide, a GLP-1 RA and/or a glucagon RA prepared according to example C11.**

| Comp osition | Pramlintide (mg/mL) | GLP-1 RA (µg/mL) | Glucagon RA (mg/mL) | Molar ratio glucagon RA / GLP-1 RA | DMSO (% v/v) | Hydroch loric acid (mM) |
|---|---|---|---|---|---|---|
| C11-1 | 1.5 | Exenatide (200) | 0 | | 67 | 7 |
| C11-2 | 1.5 | Lixisenatide (670) | 0 | | 67 | 7 |
| C11-3 | 0 | Exenatide (100) | Human glucagon (1) | 12 | 67 | 7 |
| C11-4 | 0 | Lixisenatide (170) | Human glucagon (1.5) | 12.3 | 67 | 7 |
| C11-5 | 0 | Exenatide (100) | Dasiglucagon (1) | 12.4 | 67 | 7 |
| C11-6 | 0 | Lixisenatide (330) | Dasiglucagon (1) | 4.4 | 67 | 7 |
| C11-7 | 1.5 | 0 | Glucagon (2) | | 67 | 7 |
| C11-8 | 1.5 | 0 | Dasiglucagon (1) | | 67 | 7 |
| C11-9 | 1.5 | Exenatide (200) | Glucagon (2) | 12.1 | 67 | 7 |
| C11-10 | 1.5 | Lixisenatide (330) | Human glucagon (2) | 8.5 | 67 | 7 |
| C11-11 | 1.5 | Exenatide (200) | Dasiglucagon (1) | 6.2 | 67 | 7 |
| C11-12 | 1.5 | Lixisenatide (330) | Dasiglucagon (0.5) | 2.2 | 67 | 7 |

Compositions according to the invention allow the formation of a co-formulation of pramlintide, exenatide or lixisenatide, and recombinant human glucagon or dasiglucagon in an aprotic polar solvent system.

### 3 - Compositions comprising a copolyamino acid, a glucagon RA and a GLP-1 RA.

### Example C12: Compositions comprising a copolyamino acid, glucagon and exenatide at neutral pH

A solution of 5 mg/mL L-methionine was prepared using L-methionine as powder, water was added and the pH was not adjusted.

A solution of 1 mg/mL L-methionine was prepared using 5 mg/mL L-methionine stock solution, water was added and the pH was not adjusted.

A stock solution of GLP-1 RA was prepared using GLP-1 RA as powder, 1 mg/mL L-methionine solution was added and the pH was not adjusted.

A solution of 2 mg/mL L-methionine and 0.006 N hydrochloric acid was prepared using 5 mg/mL L-methionine stock solution, water and hydrochloric acid were added and the pH was not adjusted.

A 4 mg/mL stock solution of glucagon was prepared using glucagon as powder, solution of 2 mg/mL L-methionine and 0.006 N hydrochloric acid was added and the pH was not adjusted.

A 2-fold concentrated GLP1-RA /excipients solution was prepared. In a container, stock solutions of excipients were added to water: m-cresol, glycerol, and phosphate buffer. The concentrations of m-cresol, glycerol and phosphate buffer were chosen to reach twice the desired concentrations in the compositions described in table 7. Then was added the GLP1-RA stock solution.

To a lyophilizate of copolyamino acid, 2-fold concentrated GLP1-RA /excipients stock solution was added. After complete dissolution of the lyophilizate, glucagon stock solution was added. The pH was adjusted to 7.2 by addition of a solution of hydrochloric acid or sodium hydroxide stock solutions.

The obtained compositions are detailed in table 7. All solutions contain 250 mM of glycerol, 27 mM of m-cresol, 2 mM of phosphate buffer and 1 mg/mL of L-methionine. The pH is adjusted to 7.2.

**Table 7: Compositions comprising a copolyamino acid, exenatide or lixisenatide and human glucagon prepared according to example C12**

| Composition | GLP1-RA (µg/mL) | Human glucagon (mg/mL) | Molar ratio glucagon RA / GLP-1 RA | Copolyamino acid (mg/mL) |
|---|---|---|---|---|
| C12-1 | Exenatide (145) | 2 | 16.6 | B1 (7.8) |
| C12-2 | Exenatide (145) | 2 | 16.6 | B1 (9.1) |
| C12-3 | Exenatide (145) | 2 | 16.6 | B10 (11.3) |
| C12-4 | Exenatide (145) | 2 | 16.6 | B8 (7.3) |
| C12-5 | Lixisenatide (240) | 2 | 11.6 | B1 (7.12) |
| C12-6 | Lixisenatide (240) | 2 | 11.6 | B10 (6.4) |
| C12-7 | Lixisenatide (240) | 2 | 11.6 | B8 (4.12) |
| C12-8 | Lixisenatide (240) | 2 | 11.6 | B8 (6.4) |

Compositions according to the invention allow the formation of a co-formulation of copolyamino acid with exenatide or lixisenatide, and recombinant human glucagon.

## Claims

1. Composition comprising at least two short-acting peptides chosen in at least two of the following groups, short-acting GLP-1 RA, short-acting Amylin RA and short-acting Glucagon RA.

2. Composition according to claim 1, for use as a medicament.

3. Composition according to claim 1 or 2, for use in a method of preventing or treating overweight, obesity, and related diseases and/or neurodegenerative diseases and /or Type 2 Diabetes.

4. Composition according to any of claims 1 to 3, wherein the composition is administered in a therapeutical scheme that comprises modulation of the delivered dose, such as increasing and decreasing the delivery, or even stop and restart periods.

5. Composition according to any of claims 1 to 4, wherein it comprises one short-acting GLP-1 RA and one short-acting amylin RA.

6. Composition according to any of claims 1 to 5, wherein it comprises one short-acting GLP-1 RA and one short-acting glucagon RA.

7. Composition according to claim 6, wherein the molar ratio of short-acting GLP-1 RA and short-acting glucagon RA at steady state is comprised betweeen 0.1 to 15

8. Composition according to any of claims 1 to 7, wherein it comprises one short-acting amylin RA and one short-acting glucagon RA.

9. Composition according to any of claims 1 to 8, wherein it comprises one short-acting GLP-1 RA, one short-acting amylin RA and one short-acting glucagon RA.

10. Composition according to any of the claims 1 to 9, wherein GLP-1 receptor agonist (GLP-1 RA), is chosen amongst exenatide and lixisenatide.

11. Composition according to any of the claims 1 to 10, wherein short-acting amylin RA is pramlintide.

12. Composition according to any of the claims 1 to 11, wherein short-acting glucagon RA is chosen amongst human glucagon and dasiglucagon.

13. Composition according to any of claims 1 to 12, wherein it further comprises a copolyamino acid bearing carboxylate charges and hydrophobic radicals Hy.

14. Composition according to any of claims 3 to 10, for use in a method of treating obesity, so as to diminish the food intake, **characterised in that** the composition is administered in a therapeutical scheme that comprises modulation of the delivered dose.

15. Composition according to any of claims 1 to 12, for use in a method of treating obesity, so as to diminish the food intake or increase energy expenditure, **characterised in that** the composition is administered in a therapeutical scheme that comprises stop and restart periods.

16. Composition according to any of claims 1 to 12, for use in a method of treating obesity, so as to diminish the food intake or increase energy expenditure, **characterised in that** the composition is administered in a therapeutical scheme that comprises modulation periods that allow a modulation of the rate of delivery intraday.

17. Administering device comprising:
- a composition (1) comprising at least two short-acting peptides chosen in at least two of the following groups, GLP-1 RA, Amylin RA and Glucagon RA, according to any of the preceding claims,
- an administering apparatus (2) for delivering the composition (1) to a patient, said administering apparatus (2) including:
- a reservoir (22) containing the composition (1),
- a drive mechanism (23) for pumping the composition (1) contained in the reservoir (22),
- a control unit (24) for controlling the drive mechanism (23) according to an injection profile defining at least one rate for the administering of the composition (1) to the patient,
- an injection set (26) configured to be attached to an infusion site on the patient for the subcutaneous administering of the composition (1) to the patient.
